# EUROPEAN PATENT SPECIFICATION

(11) **EP 4 228 565 B1**
(45) Date of publication and mention of the grant of the patent: **03.12.2025**
(21) Application number: 21879651.4
(22) Date of filing: 05.10.2021
(51) Int. Cl.: A61F 5/445, A61F 5/44

(54) **OSTOMY DEVICES**
OSTOMIEVORRICHTUNGEN
DISPOSITIFS DE STOMIE

(30) Priority: 14.10.2020 US 202063091419 P
(43) Date of publication of application: 23.08.2023
(73) Proprietor: Stomtop Ltd., 7414003 Ness Ziona (IL)
(72) Inventor: EYAL, Dror, 7403319 Ness Ziona (IL); EYAL, Nurit, 7403319 Ness Ziona (IL)
(74) Representative: ABG Intellectual Property Law, S.L.
(86) International application number: PCT/IL2021/051193
(87) International publication number: WO 2022/079708

(56) References cited:
- EP-B1- 2 157 944
- WO-A1-97/06757
- US-A- 6 039 714
- US-A- 6 050 983
- US-A1- 2003 028 237
- US-A1- 2004 039 357
- US-A1- 2006 052 752
- US-A1- 2012 143 155
- US-A1- 2018 221 194
- US-A1- 2020 093 633
- US-A1- 2020 155 338
- US-B1- 6 485 476
- US-B1- 8 016 802

## Description

### FIELD OF THE INVENTION

The present invention relates to novel ostomy devices and medical applications thereof. Document US 2018/221194 A1 discloses the features of the preamble of claim 1.

### BACKGROUND

An ostomy procedure needs to be carried out when the intestine of a patient losses its normal function by which waste material, i.e. stool, is discharged from the body *via* the anus. This may happen, e.g., when the intestine of a patient is affected by disease (e.g. colon cancer, bladder cancer, Crohn, Colitis) or trauma (e.g. serious abdominal injury) leading to intestinal surgery.

An ostomy procedure replaces the loss of the normal waste elimination function. In an ostomy procedure, the large intestine or the small intestine of a patient (depending upon the reason for the ostomy procedure) is cut, and the cut-end of the intestine is then drawn through an incision made in the abdominal wall of the patient, and is sutured to the skin on the outside of the incision to form a stoma. Intestinal waste, i.e. stool (or urine), can then exit the patient's body *via* the stoma, which replaces the function of the anus (or urethra) to discharge stool (or urine).

When intestinal waste exits the large intestine, the procedure is known as a colostomy and when it exits the small intestine, the procedure is known as an ileostomy. When the ostomy procedure is performed to create the stoma using the urinary system, the procedure is known as an urostomy.

Whilst the stoma provides an opening to allow waste to exit the body, the stoma has none of the functions of the anus to control the exit of waste. Thus, after an ostomy has been performed on a person, waste material freely exits the intestine through the stoma. Waste exits involuntary so the person has no control over the process. Consequently, once an ostomy has been performed on a person, it is necessary that provision is made to cater for the loss of the normal waste elimination function.

Following an ostomy procedure, it is mandatory that an ostomy-bag is used to collect the intestinal waste that exits from the stoma. The opening of the ostomy-bag is attached by an adhesive baseplate adhered directly to the patients' skin. A person living with a stoma (ostomate) must empty and replace the ostomy-bag when necessary. The frequency of replacement of the ostomy-baseplate varies, though is often every 2-3 days. Replacement of the ostomy-baseplate requires that the adhesive attachment to the skin is broken, the existing baseplate removed. Then, the person must clean the stoma and the skin around it before attaching a new ostomy-baseplate to the skin around the stoma using an adhesive.

All devices used in ostomy care are barrier sealed baseplates attached to the skin by adhesive. Typically, a layer of sealing barrier is applied around the stoma, followed by a baseplate (also known as wafer) on top of the stoma. However, products available on the market tend to have many problems.

The main deficiency of current stoma care devices is secretions leakages. This means that the stomal output containing aggressive, enzymatic fluids enters underneath the adhesive of the baseplate and leading to adhesion failure. In addition to shame, discomfort and embarrassment, the leakage causes skin irritation, wounds, infection, and necrosis of stoma and peristomal skin.

Secretions (stool or urine) leakages occur due to, e.g., improper stoma device attachment due to irregular abdomen skin around the stoma having scars and wrinkles, intensive sweat, temporary skin crease from tight garment, body position or physical and sport activities, etc. About 76% of the ostomates had experienced leakage, 91% are worried about leakage and 12% socially isolate themselves.

Sudden leakage of secretions and presence of odor are of highest concern for people dependent on a stoma appliance. In case of irregular stoma or irregular skin around the stoma, which might cause attachment-impairment of a baseplate, leakage is much more likely to happen. In addition, common stoma irregularities are prolapsed or retracted stomas. When stoma begins to retract or prolapse, the circular shape becomes irregular, the aperture of the stoma moves to the periphery and sinks into the abdomen and peristomal skin become irregular with scars and folds. Applying a standard baseplate around such a stoma would leave the peristomal skin area around the stoma uncovered and thereby exposed to the output from the stoma. Moreover, in some cases, the stoma is retracted so much that it is not even possible for it to extend or protrude through the passage in the baseplate. This means that the risk of leakage is increased.

Typical skin irregularities are inward or outward skin, scars, folds, wounds, irritation, etc. Less than 45% of ostomates have regular peristomal skin. About 75% of ostomates have experienced skin issues, and 85% of ostomates who often experience leakage have skin issues. It is especially important to break the potential vicious circle of leakage and skin erosion, which may lead to more serious peristomal skin problems.

Known stoma device have many other problems, such as: (1) the constant adhesion of the baseplate to the skin causes skin irritations, allergies, rashes, itching and pealing skin. It is impossible to shift the baseplate in order to relief such symptoms or manage leaks and alike. The only way to comfort is to replace the baseplate; (2) stool blockage - usually near the inner belly wall, stoma stenosis and stoma retraction; (3) stoma-bag wearers are constantly confronted with the unpleasant odor that is attendant to having a stoma for discharge of intestinal waste and embarrassing noises caused by intestinal gas squirt. Intestinal gas also causes bag ballooning- 91% of ostomates had experienced ballooning, and 94% worried about it; (4) the requirement of frequent bag changing; and (5) the application of a standard baseplate in irregular stoma or skin conditions is messy, difficult and takes a long time (up to one hour) because the user is required to stay without movement until the adhesive dries and the device is properly tightened.

Several alternative internal stoma devices have been designed such as those described in US 2013/0030397, US 2014/0052085 and US 2015/0057626. However, these devices are implants that require surgical intervention for installation and may not be easily removed in case of need.

Notwithstanding the various efforts directed towards developing ostomy compositions that may provide useful sealing for stoma-bags, there remains a need to provide a more reliable and functional product which can minimize leakage upon application to the skin and mitigate all issues and drawbacks described above.

Ostomates can suffer tremendous hardship. In addition to the above problems and inconvenience of dealing with their condition, ostomates often suffer from deterioration in their personal, family, social and employment relationships. This can lead to other problems and conditions, including loss of self-esteem, social isolation, and depression. The quality of life-score for ostomates who experience skin issues is only 51 opposed to 62 for those without skin issues. 40% of ostomates wake up at night, 30% limit their physical and social activities, and for 12% concerns about leakage led to the unfortunate consequence of isolating themselves. Only 19% do not feel their concerns influenced their daily life.

Accordingly, a need exists for a device that addresses the above and other issues and their consequent effects. Additional advantages, features and objects of the invention will become apparent from the detailed description below.

### SUMMARY

In a first aspect, the present invention provides an ostomy draining conduit (101) as defined in claim 1.

In a second aspect, the present invention provides an ostomy draining device comprising the above conduit (101).

### BRIEF DESCRIPTION OF THE DRAWINGS

**Fig. 1** is an illustration of an ostomy disposing/draining device according to some embodiments of the invention.
**Fig. 2** is an enlarger view of the ostomy draining device of Fig. 1.
**Fig. 3** is an illustration of one embodiment of an ostomy draining device according to the invention where the sealing sleeve is detachable from the conduit.
**Fig. 4** is an illustration of a gas absorbance filter component of an ostomy draining device according to some embodiments of the invention
**Fig. 5** is an illustration of some of the possible components, e.g. for a bag washing, of an ostomy draining device according to some embodiments of the invention.
**Fig. 6** is a 3-Dimentional cross-view of an embodiment of an ostomy draining device according to the invention where the internal sealing edge is made of a polymeric membrane anchored by a conduit in a connecting sleeve and with adjustable length configuration.
**Figs. 7A-7D** are illustrations of different possible designs and combinations of curved and linear sections in different lengths, angles, and diameters of the self-expanding internal sealing edge, some with O-rings in different locations to improve sealing capabilities of the ostomy draining device of the invention.
**Fig. 8** is a 3-Dimentional cross-view of an ostomy draining device according to the invention with adjustable length and external polymeric elastomeric membrane applied in a stoma.
**Figs. 9A-9D** are illustrations of a 3-pieces polymeric ostomy draining device of the invention having an insert conduit, external elastomeric membrane **(****Fig. 9A****),** and baseplate with bag flange **(****Fig. 9C****).** The insert and external elastomeric integration is illustrated in **Fig. 9B** and the complete device in **Fig. 9D****.**
**Figs. 10A-10B** are illustrations of two possible designs of the external elastomeric membrane of the ostomy draining device of the invention.
**Figs. 11A-11E** are illustrations of different possible designs and combinations of the self-expanding internal sealing edge of the conduit of the invention, illustrated within a device with a fixed length and an integrated external membrane: **Fig. 11A** with linear horizontal and vertical section; **Fig. 11B** with inverted seals; **Fig. 11C** with a narrow angle convex with no linear section; **Fig. 11D** with a collapsible tube; and **Fig. 11E** with a narrow angle concaved with linear vertical section, and wide angle convex with linear vertical section.
**Figs. 12A-12D** are illustrations of different possible designs and combinations of the self-expanding internal sealing edge of the conduit of the invention where the device is a fixed length: with integrated external membrane with long tube moderate and moderate angle large diameter seal **(****Fig. 12A****),** long tube with enforcement ring and sharp angle small diameter seal **(****Fig. 12B****),** Long tube and wide angle short small diameter seal **(****Fig. 12C****),** and short tube with enforcement ring and wide angle large diameter short seal **(****Fig. 12D****).**
**Figs. 13A-13C** are 3-Dimentional illustrations of an ostomy draining device of the invention with its applicator ready for application **(****Fig. 13A****),** the applicator is semi-extracted and its collapsible section is exposed **(****Fig. 13B****),** and the applicator is out of the device **(****Fig. 13C****).**
**Figs. 14A-14E** are illustration and pictures of an ostomy draining device of the invention with a balloon configuration: **Figs. 14B-14C****:** devices made of silicone and the balloon configuration is inverted proximal edge **(****Fig. 14B****)** or the distal edge **(****Fig. 14C);** and **Fig. 14D****:** devices made of polyurethane and the balloon configuration is inverted proximal edge (left) and distal edge (right).
**Figs. 15A-15B** are pictures of different designs of the sealing edge of the ostomy draining device of the invention made of silicone **(****Fig. 15A****)** or polyurethane **(****Fig. 15B****).**
**Fig. 16** is a picture of an ostomy draining device of the invention made of silicone, used for animal *in-vivo* study, ready for insertion into the stoma using a Punch-Down fold method.
**Figs. 17A-17B** are pictures of two ostomy draining devices of the invention made of silicone, used for animal *in-vivo* study. **Fig. 17A****:** a device made of high shore A silicone, 40mm long, large tube diameter, narrow external membrane and rigid baseplate; and **Fig. 17A****:** a device made of low shore A silicone, 80mm long, small tube diameter and wide external membrane integrated with baseplate.
**Figs. 18A-18F** are pictures of animal *in-vivo* study conducted with the ostomy draining device of the invention showing the stoma **(****Figs. 18A & 18D****),** the device applied in the stoma **(****Figs. 18B & 18E****)** and an endoscopy image of the device **Figs. 18C & 18F** in the stoma in the first week **(****Fig. 18A-C****)** and the fourth week **(****Fig. 18D-F****)** of the study.

### DETAILED DESCRIPTION

Persons living with a stoma (ostomates) must replace an ostomy-baseplate on average every 2 days. This procedure is time consuming, mainly due to the time it takes to clean and dry the area around the stoma, adhere the new baseplate and wait until the baseplate will be adhered properly. In addition, due to often leakages, ostomates use supportive accessories such as special rings and patches and protective pastes and sprays that need additional operative time. The replacement of the current baseplates requires clean and isolated room and the availability of the accessories. Therefore, most of the replacements are done in the ostomate's home. That mean that when there is a leak, the patient must go back home and stop his daily activity. Additionally, ostomates tend to suffer from many problems, such as irritated skin around the stoma, leakage of secretions, various problems associated with ostomy-bag replacement, etc. The present invention thus provides an ostomy disposing device which is fast and easy to replace, prevent leakages, there is no need in adhesion and it is not dependent on skin condition and abdomen structure. The new disposing device overcomes all the above problems and others.

The term "ostomate" as used herein refers to any individual/patient that needs an alternative way to discharge waste- whether stool via the large- or small-intestine, or urine from the urinary system. Accordingly, the ostomy draining conduit (101) and the ostomy draining device according to any of the embodiments herein is for ileostomy-, colostomy-, or urostomy- draining.

The present invention thus provides a disposable ostomy draining device.

Accordingly, in a first aspect, the present invention provides an ostomy draining/disposing conduit (101) for an ostomate (ostomy patient) comprising a proximal self-expanding internal sealing edge (111) designed to be inserted into a stoma and sealing passage between the exterior wall of the conduit and the interior wall of the stoma; and a distal external draining edge, such that when inserted into the stoma it allows passage of liquid and stool while preventing passage between the conduit's exterior wall and the stoma's interior wall, characterized in that: (i) the proximal self-expanding internal sealing edge's (111) diameter is smaller than an intestine's diameter, and (ii) the proximal self-expanding internal sealing edge (111) is self-collapsible, such that it can be modified/reshaped for insertion thereof into the stoma, and return to its original shape once inserted into the stoma.

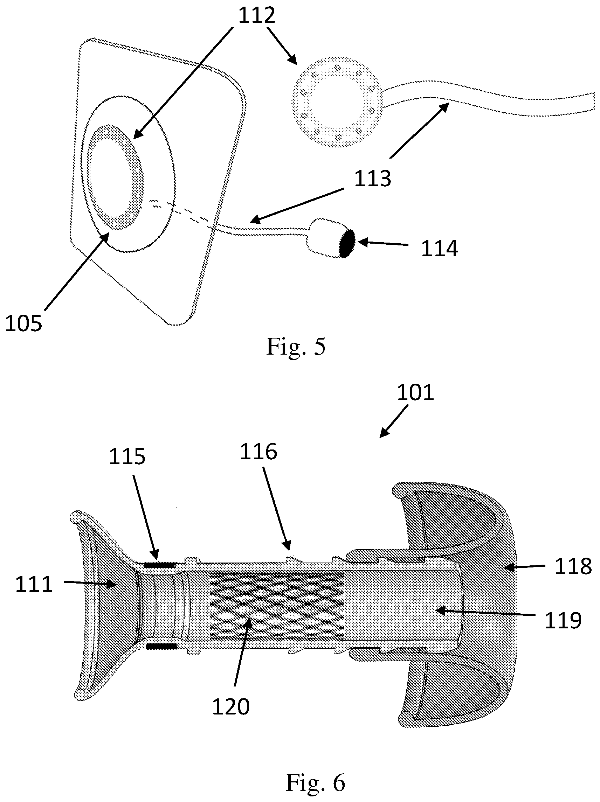

In specific embodiments thereof, the ostomy draining conduit (101) further comprises a hollow connecting sleeve (119) connecting the proximal self-expanding internal sealing edge (111) and said distal external draining edge, characterized in that the sleeve's length is longer than the abdomen's wall (abdomen's thickness). A support stent (120) is inserted into the sleeve (119) - see **Fig. 6** to assist in anchoring the sleeve in the stoma and to improve sealing, such that the proximal self-expanding internal sealing edge (111) is placed within the stoma at the inside-part of its opening, while allowing the distal external draining edge to extend outside the stoma thereby enabling connecting thereof to a stoma-bag for collection of, e.g., stool or urine: the support stent (120) is designed to apply radial force/pressure of the sleeve's (119) external walls against the inner walls of the stoma, which to anchor the conduit's sealing edge (111) tight to abdomen's internal wall and improve sealing capabilities.

In certain embodiments, the ostomy draining conduit (101) with the sleeve (119), further comprises: a flexible/elastic sealing sleeve/adaptor/membrane/cap (118) mounted onto said connecting sleeve (119), wherein movement of said external membrane/cap (118) along the sleeve (119) towards the internal sealing edge (111) creates a sealing axial force that affix the conduit (101) in place, and wherein said external membrane/cap (118) is anchored along the sleeve (119) by dedicated protrusions and rims (116), which interlock with one another to prevent unintentional dissociation of the external membrane/cap (118) from the sleeve (119). This enables adjustment of the length of the conduit to the abdomen's thickness and aids in preventing movement of the conduit (101) when inserted into the stoma and in preventing leakage due to movement of the conduit inside the stoma. This further improves sealing capabilities of the conduit: when moving the elastic sealing adaptor (118) along the sleeve (119), an axial force is applied on the internal sealing edge (111) pressing it against the stoma's inner wall/opening, thereby sealing the passage between the conduit's external walls and the stoma's inner walls.

Accordingly, in certain embodiments, the ostomy draining conduit (101) according to any of the embodiments above, comprises both a support stent (120) and a flexible/elastic sealing sleeve/adaptor/membrane/cap (118) mounted onto said connecting sleeve (119), thereby enabling applying both axial and radial forces/pressures to facilitate sealing and anchoring capabilities of the conduit - see Fig. 6.

In certain embodiments of the ostomy draining conduit (101) according to any of the embodiments above, the connecting sleeve (119) is or comprises a region that is axially self-collapsible (131), thereby preventing unintentional damage to the stoma's walls during insertion of the conduit into the stoma due to applying unintentional high force that might cause damage to the tissue while inserting the conduit.

In certain embodiments, the length of the ostomy draining conduit (101) according to any of the embodiments above is adjustable, thereby enabling adjusting its length according to the abdomen's thickness. In specific embodiments, the adjustment of the conduit's length is by movement of the external membrane/cap (118) along the sleeve (119) and anchoring thereof by said dedicated protrusions and rims (116).

In certain embodiments of the ostomy draining conduit (101) according to any of the embodiments above, the proximal self-expanding internal sealing edge (111) is radially self-collapsible, characterized in that it can be modified/re-shaped for insertion thereof into the stoma by enforcing it axially into the stoma, and return to its original shape once inserted into the stoma. In alternative or added embodiments, the conduit (101) is balloon expandable, meaning that once inserted into the stoma, the proximal self-expanding internal sealing edge (111) is inflated to: prevent leakage; assist in the anchoring of the conduit (101) in place and prevent its unintentional extraction from the stoma; and adjust the length of the conduit to fit the abdomen's thickness. When such a balloon is used, the conduit can be removed from the stoma by simply deflating the balloon. The conduit (101) can be provided in crimped form or can be crimped immediately prior to its insertion into the stoma.

In certain embodiments, the conduit (101) according to any of the embodiments above and/or its self-expanding internal sealing edge (111), is self-collapsing by enforcing it axially into the stoma to allow the application/insertion thereof with no in-site pre-use crimping. This means that the mere pressure applied on the conduit (101) during insertion into the stoma is sufficient for it to collapse inwardly to allow its insertion into the stoma.

In further embodiments, the conduit (101) according to any of the embodiments above, is designed to be removed from the stoma by simply pulling it outwardly. In specific alternative embodiments, the conduit (101) is removed from the stoma by a conduit extractor consisting essentially entirely of a flexible polymeric rod with springy hooks to grasp the inner end of the conduit and crimp it by stretching the inner end further inside to avoid intestinal erosion during conduit pullout.

In certain embodiments, the conduit's diameter is smaller than the intestine's diameter to avoid any risk or damage to the intestine's wall and intestine's tissue during insertion therein. In further embodiments, the diameter of the self-expanding internal sealing edge (111) is also smaller than the intestine's diameter to avoid any risk or damage to the intestine's wall and intestine's tissue when the conduit resides within the stoma, wherein the external diameter of the self-expanding internal sealing edge (111) is larger than the diameter of the stoma's passage in the abdomen's internal wall to enable anchoring the conduit in the stoma, provide adequate sealing, and prevent unintentional extraction of the conduit from the stoma.

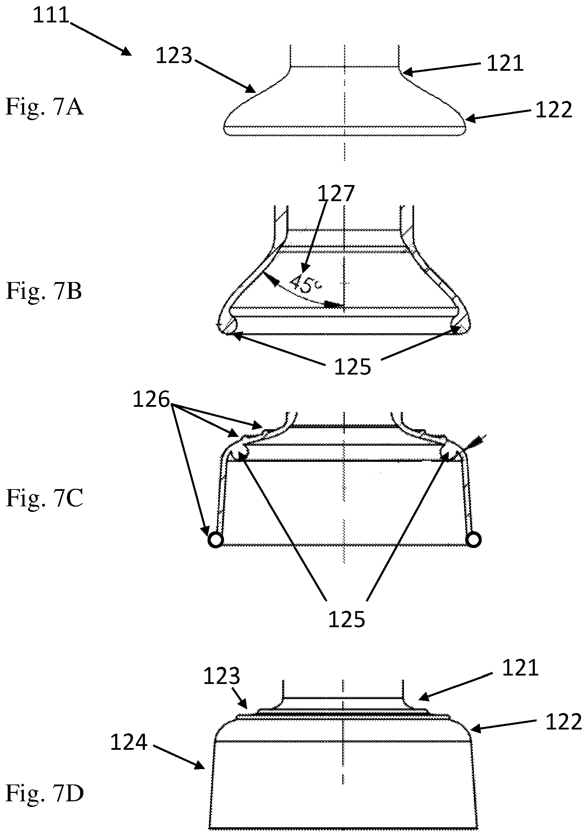

In certain embodiments of the conduit (101) according to any of the embodiments above, the profile of the proximal self-expanding internal sealing edge (111) is designed as a combination of all or part of (see e.g., **Fig. 7****):** an outward radial section (121), an inward radial section (122), a horizontal linear section (123), and a vertical linear section (124), to enhance its self-collapsibility and minimize the pressure applied on the intestine wall, while maintaining its sealing efficacy.

In certain embodiments of the conduit (101) according to any of the embodiments above, the radius of the outward and inward radial sections (121, 122) is: from 0.5 to 5mm; from 0.75 to 5mm; from 1 to 5mm; from 1.5 to 5mm; from 2 to 5mm; from 2.5 to 5mm; from 0.5 to 4.5mm; from 0.5 to 4mm; from 0.5 to 3.5mm; from 0.5 to 3mm; and from 1.5 to 3.5mm. In certain embodiments, its thickness is: from 0.2 to 2mm; from 0.3 to 2mm; from 0.4 to 2mm; from 0.5 to 2mm; from 0.6 to 2mm; from 0.7 to 2mm; from 0.8 to 2mm; from 0.9 to 2mm; from 0.2 to 1.9mm; from 0.2 to 1.8mm; from 0.2 to 1.5mm; from 0.2 to 1.2mm; from 0.3 to 1.2mm; from 0.2 to 1mm; or from 0.3 to 0.9mm. In further embodiments, the length of the radial section is: from 0.2 to 7mm; from 0.3 to 7mm; from 0.4 to 7mm; from 0.5 to 7mm; from 0.2 to 6mm; from 0.2 to 5mm; from 0.2 to 4mm; from 0.2 to 3mm; from 0.4 to 3mm; or from 0.5 to 2.5mm. The angle (127) of the horizontal linear section is 89° from to 25°, preferably 65° to 35°, its thickness is from 0.2 to 2mm, preferably 0.3-0.9mm, and the length of the horizontal linear section is from 2 to 12mm, preferably 4-9mm. The angle (127) of the vertical linear section is from 20° to 1°, preferably 7° to 2°, its thickness is from 0.2 to 2mm, preferably 0.3-0.9mm, and the length of the vertical linear section is from 2 to 12mm, preferably 6-10mm.

In certain embodiments of the conduit (101) according to any of the embodiments above, the proximal self-expanding internal sealing edge (111) is equipped with one or more O-rings (125,126) to improve and facilitate its self-expandability and sealing efficacy. The O-rings (125,126) may be located at one or more of the linear or curved sections of the sealing edge (111). The O-rings may be external (126) to improve sealing in case of irregular intestine profile, or internal (125), to minimize high point pressure on the intestine wall. In specific embodiments, the O-rings' radius is: from 0.2 to 1.5mm; from 0.3 to 1.5mm; from 0.4 to 1.5mm; from 0.5 to 1.5mm; from 0.2 to 1.4mm; from 0.2 to 1.3mm; from 0.2 to 1.2mm; from 0.2 to 1.1mm; from 0.2 to 1mm; from 0.3 to 1.4mm; from 0.4 to 1.3mm; from 0.4 to 1.2mm; or from 0.5 to 1mm.

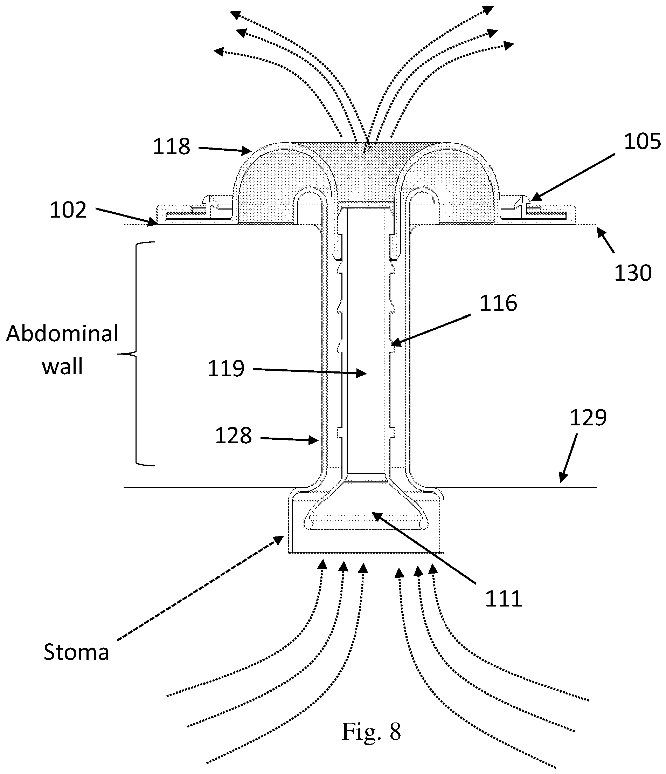

The conduit (101) according to any of the embodiments above is designed for easy and simple insertion into a stoma, anchoring in place, and prevent unintentional removal therefrom, while avoiding undesired leakage therethrough. For instance, in certain embodiments, after insertion of the conduit (101) into the stoma, the external draining edge is anchored/maintained in place using an elastic sealing external membrane (118) (see illustrated in **Figs. 6** **&** **8****)** mounted onto the connecting sleeve (119), moved towards the edge (111) while creating a sealing axial force and affixed in place by rims (116), e.g. on the exterior surface of the sleeve (119). In alterative or additional embodiments, the anchoring and sealing axial force is applied by a self-expandable support stent (120) located at the connecting sleeve (119) designed to, e.g., maintain the sleeve (119) in an open state and allow passage of, e.g., stool therethrough; and apply and maintain pressure of the sleeve's outer walls against the stoma's inner walls thereby aiding in leakage prevention. The movement of the elastic sealing external membrane (118) along the connecting sleeve (119) also enables adjustment of the length of the conduit to fit any abdomen's thickness.

In certain embodiments, the conduit (101) according to any of the embodiments above, is a self-expandable conduit, i.e. with a self-expanding internal sealing edge (111) for sealing the passage between the exterior wall of the conduit and the interior wall of the stoma. In further embodiments, the conduit (101) is designed to be removed from the stoma by simply pulling it outwardly. In specific alternative embodiments, the conduit (101) is removed from the stoma by a conduit extractor consisting essentially entirely of a flexible polymeric rod with springy hooks to grasp the inner end of the conduit and crimp it by stretching the inner end further inside to avoid intestinal erosion during conduit pullout.

In certain embodiments, the conduit (101) according to any of the embodiments above, further comprises one or more reinforcement rigid rings (115) designed to prevent axial collapsing and flipping of the expanding internal sealing edge (111) during insertion thereof into the stoma.

In certain embodiments, the conduit (101) according to any of the embodiments above, has constant or alternating surface area sections applying lower radial force on the intestine internal wall/stoma's opening constantly or alternately to, e.g., enable temporary relief and avoid constant pressure on the intestinal wall/stoma's opening and mucosa that may cause erosion, ischemia, perforation and necrosis. This enables placing the conduit inside a stoma for a prolong period of time without causing damage to the tissues (the intestine, the stoma and surrounding skin) and without causing discomfort or irritation to the user.

In certain embodiments of the conduit (101) according to any of the embodiments above, the conduit's length is of 40 to 120mm; 50 to 120mm; 60 to 120mm; 70 to 120mm; 80 to 120mm; 90 to 120mm; 100 to 120mm; 50 to 110mm; 50 to 100mm; 50 to 90mm; 60 to 110mm; 70 to 100mm; or 80 to 110mm, the conduit's expanded diameter is of: 2.5-50mm; 3.5-50mm; 5-50mm; 7.5-50mm; 10-50mm; 2.5-45mm; 5-45mm; 7.5-45mm; 10-45mm; 2.5-40mm; 5-40mm; 7.5-40mm; 10-40mm; 2.5-35mm; 5-35mm; 7.5-35mm; 10-35mm; 2.5-30mm; 5-30mm; 7.5-30mm; 10-30mm; 2.5-25mm; 5-25mm; 7.5-25mm; 10-25mm; 10-20mm; 15-50mm; 20-50mm; 25-50mm; 30-50mm; or 35-50mm; wherein the conduit's length and diameter are dependent on (a) the ostomy type (colostomy / ileostomy / urostomy); and (b) the ostomate's personal dimensions. In further specific embodiments, the conduit's wall thickness is of 0.05-1.00mm.

In a second aspect, the present invention provides an ostomy draining device for an ostomate comprising the conduit (101) according to any of the embodiments above. In specific embodiments, the conduit (101) comprises a proximal self-expanding internal sealing edge (111) for insertion into a stoma for sealing passage between the exterior wall of the conduit and the interior wall of the stoma, and an external draining edge.

In specific embodiments of the ostomy draining device of the invention, the conduit (101) further comprises a hollow sleeve (119) connecting the proximal self-expanding internal sealing edge (111) and the distal external draining edge, characterized in that the sleeve's length is longer than the abdomen's thickness/ abdomen's wall.

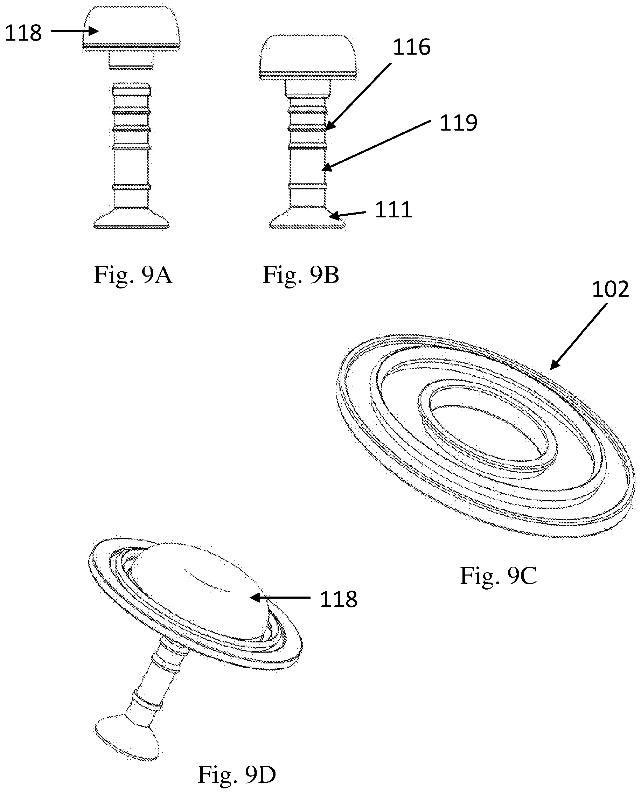

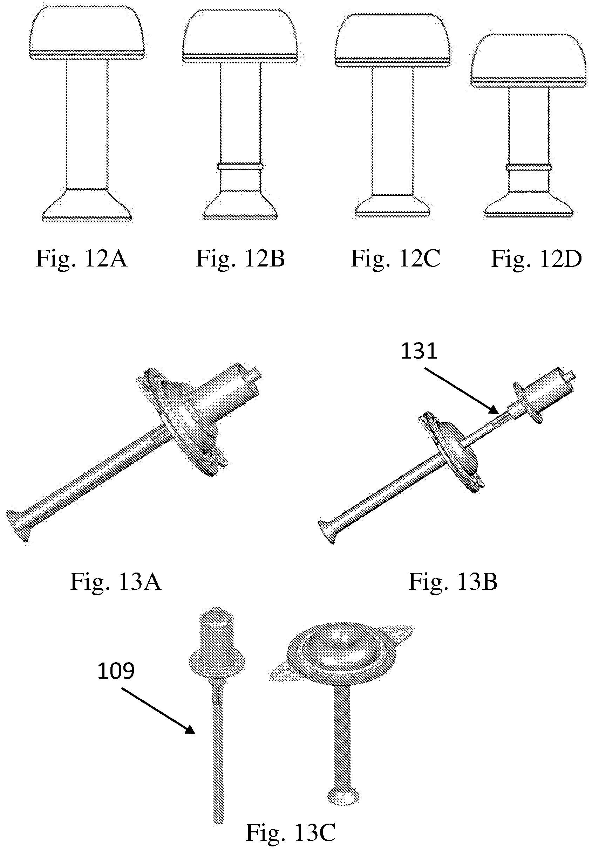

The sleeve (119) enables the insertion of the conduit (101) into the stoma such that the proximal self-expanding internal sealing edge (111) is placed within the stoma at the inside-part of its opening, while allowing the distal external draining edge to extend outside the stoma, which enables connecting same to a stoma-bag. The length of the sleeve (119) can be modified or fabricated in advance according to the thickness of the patient's abdomen, which is influenced by the amount of fat layers, muscles, and other tissues in the stoma's area. **Fig. 9** illustrates how the length of the sleeve can be adjusted/shortened by moving an elastic sealing external membrane/cap (118) along the sleeve (119) thereby adjusting the overall length of the sleeve according to the abdomen's thickness. **Figs. 12B and 12D** illustrate alternative conduits with a fixed-length sleeve, wherein the length of the sleeve is determined in advance, or is selected from multiple lengths, according to need.

In specific embodiments, the ostomy draining device of any of the embodiments above further comprises a body attachment baseplate (102) for attaching said ostomy device to an ostomate, said baseplate (102) comprising: a stoma-bag adapter flange (105) without an external stoma wall sealant, designed to surround the stoma's external wall without direct contact therewith, characterized in that said baseplate (102) comprises an adhesive-free or non-adhesive layer on the side designed to be in contact with the patient's skin.

Currently used baseplates are attached and sealed to the stoma's outer wall and are glued to the peristomal skin. If all is well, it needs to be replaced every 1-3 days to avoid damage and irritation to the skin or since the adhesion is loosed. However, in case of leakage or peristomal skin complications, it needs to be replaced even a couple of times a day. Accordingly, the present invention provides an ostomy draining device for an ostomate that may be changed in a very low frequency of only every 2-4 weeks. Moreover, even if need arises to replace the device more frequently, even several times a day, the replacing is easy, simple and does not damage the skin (which might happen when removing the baseplate with the adhesive).

Accordingly, in certain embodiments, the ostomy draining device of any of the embodiments above is suitable for use for up to four weeks without requiring changing and without causing damage to the tissues, such as skin irritations, lesions, erosion, ischemia, perforation and necrosis.

As explained above, currently used baseplates have a tight opening fitting the stoma's opening and are attached and sealed to the stoma's outer wall and are glued to the peristomal skin. Moreover, in case of irregular stoma formation, e.g. elliptic, the user has to cut the opening to fit the stoma's shape. This means that every time a patient needs to remove the baseplate, he/she needs to remove the glue, which is not pleasant, and optionally cut-adjust the opening according to the stoma's shape. Also, the tight contact prevents the skin from "breathing" which often cause skin irritations and lesions at the glued area. Contrary to that, the diameter of the present flange (105), is with a central stoma bore significantly greater than the stoma outer diameter. In certain embodiments, the adhesive-free or non-adhesive layer is a sticky layer having high-friction capabilities designed to prevent free movement of the baseplate (102) from the patient's skin, while enabling moving or detaching the baseplate (102) form the ostomate's skin while the stoma-bag is connected to the baseplate (102) while maintaining its sealing integrity.

In certain embodiments of the ostomy draining device of any of the embodiments above, the baseplate's inner layer, which is intended to be in contact with the patient's skin, is made-of or coated-with bribed sticky silicone or any other sticky high friction or high skin affinity polymer, textile, woven or nonwoven fabric. In alternative embodiments, the baseplate (102) itself is made of bribed sticky silicone or any other sticky high friction or high skin affinity polymer, textile, woven or nonwoven fabric.

In certain embodiments, the ostomy draining device of any of the embodiments above further comprises a gas filter (110) with an odor absorbance layer. In specific embodiments, when the ostomy draining device comprises a baseplate (102), the gas filter (110) may be placed between the baseplate (102) and the stoma-bag flange (105). Accordingly, in specific embodiments of the baseplate (102) according to any of the embodiments above, a hydrophobic filter layer containing gas and odor absorbance material (e.g. charcoal) is added to the bottom of the baseplate and inner peripheral circumference of the bag flange (105) to prevent occasional bad odor leaks and ballooning while preventing liquid leakage and maintaining aeration to skin below the baseplate (102).

In certain embodiments of the ostomy draining device of any of the embodiments above, the baseplate is flippable, meaning that it can be flipped even when the device is inserted into the stoma, thereby allowing access to the skin underneath the baseplate for, e.g., enabling monitoring and management of peristomal skin under the baseplate, ventilation thereof or treating if needed. Such flippability may also assist in the insertion of the device into the stoma and/or for connection thereof a stoma-bag.

In certain embodiments, the ostomy draining device of any of the embodiments above further comprises a flexible/elastic sealing sleeve/adaptor/membrane (118) integrated with the baseplate (102), such that said external draining edge of said conduit (101) enables sealed (leakage-free) fluid connection between said conduit (101) and a stoma-bag that is connected to said stoma-bag adapter flange (105). In specific embodiments, the flexible sealing sleeve (118) is welded to the stoma-bag flange connector's inner circumference.

In certain embodiments of the ostomy draining device according to any of the embodiments above, the conduit's external draining edge comprises a detachable connection flange (106), e.g., for attaching flexible sealing sleeve (118) and/or other components. In specific embodiments, the detachable connection flange (106) is equipped with a capping plug (108) that enables closing the conduit (101) and preventing stool from exiting, e.g. during bag replacement. In further specific embodiments, the flexible sealing sleeve (118) is attached to the flange (106) by a clamp or an elastic ring (107). In certain embodiments, the flexible sealing sleeve (118) is attached to the detachable connection flange (106) by a clamp or an elastic ring (107) thereby enabling easy and simple attachment and detachment from one another.

In certain embodiments, the ostomy draining device according to any of the embodiments above, further comprises a plug (108) designed to block the draining through the conduit (101) when no stoma-bag is connected to the device, thereby enabling short-term bag removal, e.g. for changing the bag, sexual relations, extreme activity, etc. When a stoma-bag is connected to the device, e.g. *via* the baseplate flange (105), the plug (108) is either manually removed or automatically opened to allow draining into the bag. In specific embodiments, the conduit's outer edge is attached to a polymeric flange (106) enabling detachable connection of the flexible sleeve adapter (118) and assembling optional elements such as a plug (108) or silencer. In further or alternative specific embodiments, the flange (106) has a concentric bore equal to the diameter of the expanded conduit (101), external diameter exceeding the bore by 2-10mm and thickness of 2-5mm. In specific embodiments, the peripheral margins of the flange (106) are engraved in order to place a sealing ring to attach the sleeve adapter (118) by a clamp or elastomeric ring (107).

In certain embodiments, the flexible sealing sleeve (118) according to any of the embodiments above, is made of a hydrophobic membrane filter permeable to intestine gas and impermeable to intestine liquid. This allows release of gas from the device when installed thereby eliminating undesired inflating of the stoma-bag attached to the device, and eliminates unpleasant sounds associated with release of such gases. In specific embodiments, the flexible sealing sleeve (118) is made-of or coated-with or associated-with a gas filter with an odor absorbance layer to obviate odor release when the gas is discharged.

In specific embodiments of the ostomy draining device according to any of the embodiments above that comprises a flexible sealing sleeve (118), the sleeve (118) is made of an elastomer providing the sleeve (118) with elastomeric capabilities. In specific embodiments thereof, the elastomeric flexible sealing sleeve (118) can stretch against the baseplate (102), thereby fixing the conduit (101) in place and making the connection with the inner-abdomen/stoma's wall airtight to assure leakage-proof attachment. In further specific embodiments, the flexible sealing sleeve (118) enables to compensate the total length of the ostomy draining device by more than 5mm while maintaining its sealing integrity, thereby allowing adjustment of the ostomy draining device's length to a variety of ostomates abdomen wall dimensions. This provides flexibility during instalment of the device and reduces the need to manufacture the device with conduits in many sizes and lengths.

In certain embodiments, the ostomy draining device according to any of the embodiments above further comprises: an elastic sealing external membrane (118) mounted onto the conduits' connecting sleeve (119), wherein movement of the external membrane (118) along the sleeve (119) towards the expanding internal sealing edge (111) creates a sealing axial force to affix the internal sealing edge (111) in place, wherein the external membrane (118) is anchored along the sleeve (119) by dedicated protrusions/rims (116) )- see illustration in **Fig. 8****.**

In specific embodiments of the ostomy draining device according to any of the embodiments above that comprises a flexible sealing sleeve (118), the flexible sealing sleeve (118) is a semi-rigid external sealing membrane (118) designed to be mounted onto the connecting sleeve (119)- see illustration in **Fig. 8****.** In such embodiments, the connecting sleeve (119) is further equipped with rims/protrusions (116) and the external sealing membrane (118) is equipped with a spline/grove (or *vise-versa*) that enable the two parts to slip axially and adjust the length of the ostomy draining device to variety of ostomates abdomen wall dimensions (129-130). In further specific embodiments, the rims (116) are designed to provide better sealing in the abdomen wall cross-section area. The rims may have a rounded shape to avoid harm to the tissue.

In specific embodiments, the flexible sleeve adapter (118) according to any of the embodiments above consists essentially entirely of a thin foil, about 20-1,000µm thick, made of polyethylene (PE), polyurethane (PU) or ethylene-vinyl acetate (EVA), welded or adhered to inner bore of the baseplate flange (105) and welded to the conduit flange peripheral. In other specific embodiments, the flexible sleeve adapter (118) is made of polymeric hydrophobic filter with 0.5-6µm pores, enabling intestine gas to exit from the stoma-bag to prevent bag ballooning while blocking liquid and stool exit. In further specific embodiments, bad odor is prevented by a gas absorbance filter (110) attached to the baseplate (102). In other specific embodiments, the flexible sealing sleeve (118) is made of an elastomer, enabling stretching it over a cylinder surrounding the stoma against the baseplate (102), such that the conduit (101) is fixed and tightened to the inner abdomen wall to assure leakage-proof attachment. In further alternative specific embodiments, the flexible sleeve adapter (118) is attached to the conduit's flange by a clamp or an elastic ring (107) to adjust the distance between the conduit flange (105) and the baseplate (102) and to enable separate and independent replacement of the conduit (101) and the baseplate (102).

In certain embodiments of the ostomy draining device according to any of the embodiments above, the conduit (101) further comprises a self-expandable support stent (120) located therein. This support stent (120) applies an axial force against the stoma's wall thereby improving anchoring and sealing capabilities of the conduit inside the stoma by, e.g., maintaining the sleeve (119) in an open state and allowing passage of, e.g., stool therethrough; and applying and maintaining pressure (constant or alternate) of the sleeve's outer walls against the stoma's inner walls thereby aiding in leakage prevention.

In certain embodiments of the ostomy draining device according to any of the embodiments above, the conduit (101) is a self-expandable conduit, i.e. with a self-expanding internal sealing edge (111) for sealing the passage between the exterior wall of the conduit and the interior wall of the stoma. Such self-expandable capabilities can be obtained by, e.g., a balloon edge, using a memory material, a crimping aid (such as an applicator), etc. Accordingly, in certain embodiments, the conduit (101) of the ostomy draining device according to any of the embodiments above is expandable: by balloon expanding; by linear shortening and shrinking of the axis length; or by rotary shortening and shrinking of the axis length; or any combination thereof. In specific embodiments, the conduit (101) is a Self-Expandable Polymeric Conduit partially or fully covered with an internal or external polymeric (e.g. polyethylene or silicone) membrane, having an inner flared sealing edge and a draining outer edge connectable to a draining reservoir (e.g. a stoma-bag).

In certain embodiments of the ostomy draining device according to any of the embodiments above, the length of the ostomy draining conduit (101) is adjustable, thereby enabling adjusting its length according to the abdomen's thickness. In specific embodiments, the adjustment of the conduit's length is by movement of the external membrane/cap (118) along the sleeve (119) and anchoring thereof by said dedicated protrusions and rims (116).

In certain embodiments, the ostomy draining device according to any of the embodiments above, comprises both a support stent (120) and a flexible/elastic sealing sleeve/adaptor/membrane/cap (118) mounted onto said connecting sleeve (119), thereby enabling applying both axial and radial forces to facilitate sealing and anchoring capabilities pf the conduit.

In certain embodiments of the ostomy draining device according to any of the embodiments above, the conduit (101) further comprises one or more reinforcement rigid rings (115), made of, e.g., polymer or metal, designed to prevent axial collapsing of the sleeve and axial flipping of the expanding internal sealing edge (111) or the conduit during insertion thereof into the stoma and to improve/facilitate the expandability of the internal sealing edge (111) in the intestine, and strengthen the expanded sealing edge (111) once in place. Such reinforcement rigid rings (115) may further assist in the leak-proof sealing of the device by applying (constant or alternate) pressure against the stoma's walls and/or opening. In specific embodiments, such reinforcement rigid rings (115) are collapsible to enable insertion of the conduit (101) into the stoma, while returning to their original shape after insertion.

In certain embodiments of the ostomy draining device according to any of the embodiments above, the connecting sleeve (119), when present, is axially collapsible to ensure axial collapsing when the ostomy draining device is accidently pushed from the outside, e.g. during exercise or an accidental hit. The collapsing of the connecting sleeve (119) neutralizes the transfer of an axial movement to the conduit (101) and prevents a possible leakage due to potential detachment of the internal sealing edge (111) from the internal abdomen wall. In alternative or additive specific embodiments, the connecting sleeve (119) or its distal external edge is made of a non-elastic polymer to enable applying a constant axial force on the conduit (101) by the external sealing membrane (118) when mounted thereon.

In certain embodiments of the ostomy draining device according to any of the embodiments above, the conduit (101), the connecting sleeve (119), and the external sealing membrane (118), are made of different rigidity (Shore A value) polymer. For example: the conduit (101) is made of moderate rigidity (e.g. 40-60A) polymer to enable high flexibility and low force application while maintaining its figure for improved sealing; the connecting sleeve (119) is made of low rigidity (i.e. less than 40A) polymer to ensure axial collapsing when the ostomy draining device is accidently pushed from the outside. The collapsing of the connecting sleeve (119) neutralizes the transfer of the axial movement to the internal sealing edge (111) of the conduit (101), and thus prevents leakage caused by the optional detachment of the internal sealing edge (111) from the internal abdomen wall, or any other possible combination of different rigidities.

In certain embodiments of the ostomy draining device according to any of the embodiments above, the conduit (101) is designed to be inserted into the stoma by an applicator (109), wherein said applicator (109) constitutes an integral detachable part of the device. In alternative or additional embodiments, prior to insertion into the stoma, the conduit (101) is crimped to assist in its insertion into the stoma without damaging the stoma's walls. The conduit can be crimped in any suitable technique, such as by using: an applicator (109) as mentioned herein, wherein the conduit (101) is crimped thereon prior to insertion; a sleeve, e.g. a flexible polymeric sleeve, which constitutes an integral detachable part of the device; or by using an inflatable balloon, or any combination thereof. In specific embodiments, the sleeve is made of degradable polymer designed to self-dissolve after insertion into the stoma within about 5 to 300 sec. thereby obviating the need for removal thereof after insertion. In alternative specific embodiments, when both a sleeve and an applicator are used, and when the sleeve is not degradable, the sleeve can be removed from the conduit (101) after insertion into the stoma by poling it with the applicator (109).

In certain embodiments of the ostomy draining device according to any of the embodiments above, the conduit (101) or its connecting sleeve (119) comprises a region or is made-of a self-collapsible material, which enables the conduit to collapse axially when too much power is applied thereon, thereby preventing unintentional damage to the stoma's walls during insertion of the conduit into the stoma due to applying unintentional high force that might cause damage to the tissue while inserting the conduit.

In specific embodiments, when the ostomy draining device of the invention comprises a detachable applicator (109), the applicator (109) is self-collapsible or comprises a self-collapsible region (131) when an axial force higher than 20N, and preferably higher than 5-10N is applied, thereby preventing causing unintentional and undesired damage to the stoma's walls, e.g. erosion or perforation, during insertion of the conduit into the stoma due to the application of excessive force against the stoma's walls when pushing the applicator/conduit inwardly. In addition or alternative embodiments, the applicator (109) is rigid enough to push the conduit into the stoma, but flexible enough to allow moving around corners and corves within the stoma during insertion therein.

The term "collapsible" as used herein with reference to the applicator means that the applicator (109) has a rigidity that enables it to push the conduit inwardly into a stoma, while being capable of collapsing when it reaches/hits the stoma wall, thereby preventing unintentional application of excessive force on the stoma wall that might damage it cause leakage of, e.g. stool, into the abdominal space (perforation).

In certain embodiments of the ostomy draining device according to any of the embodiments above, the conduit (101) is crimped by an applicator (109) that is a flexible crimping sleeve during storage, said sleeve is designed to assist in the insertion of the conduit into the stoma. In specific embodiments, the sleeve is made of a fast water soluble or biodegradable polymer (e.g. PLA, PEG5000, dextran, etc.) that is designed to dissolve or degrade rapidly when exposed to the environment of the intestine and subsequently let the conduit (101) expand and anchor in place inside the stoma. In alternative specific embodiments, the applicator (109) is a thin flexible polymeric sleeve that needs to be removed after insertion of the conduit into the stoma. In specific embodiments, such as sleeve is made of a flexible polymeric tube with rounded head in the inner end and a grip in the outer edge. The crimping polymeric sleeve is continued as a polymeric string threaded through the applicator tube bore out to the conduit flange: the conduit (101) is inserted to the stoma by the applicator (109) and once in place the crimping sleeve is removed by pooling its string, removed through the applicator bore and the conduit (101) is expanded in the proper position.

In certain embodiments, the conduit (101) according to any of the embodiments above is made of a shape-memory material that enables crimping the conduit for insertion thereof into the stoma while allowing it to return to its original shape after insertion when it resides in place. In addition, this shape-memory material enables the conduit to remain in place while preventing leakage during ordinary and even strain activity that might shift and pressure the conduit. In alternative embodiments, the conduit (101) is made of a non-shape-memory material (i.e. stainless steel or polymer). In such a case, the conduit may be crimped in any suitable technique, such as by stretching it to its full length and then expanding it by shortening the axis length. The shortening can be done by linear pushing or screw rotary motion of one conduit edge against the other.

In certain embodiments, the conduit (101) according to any of the embodiments above is crimped by stretching the conduit (101) with an applicator (109) anchored to the inner end of the conduit before conduit insertion to the stoma and releasing the anchored applicator (109) once the conduit is located in place to allow the conduit (101) to expand.

In certain embodiments of the ostomy draining device according to any of the embodiments above, the conduit (101) is self-collapsing to allow the application/insertion thereof with no in-site pre-use shrinking. The mere pressure applied on the conduit (101) during its insertion into the stoma is sufficient for it to collapse inwardly to allow its insertion. In certain embodiments, the conduit's (101) and/or the self-expanding internal sealing edge's (111) diameter is smaller than the intestine's diameter to avoid any risk or damage to the intestine's wall and intestine's tissue. Notably, the external diameter of the self-expanding internal sealing edge (111) is larger than the diameter of the stoma's passage through the abdomen wall to enable anchoring the conduit in the stoma and prevent unintentional extraction of the conduit from the stoma.

In certain embodiments of the ostomy draining device according to any of the embodiments above, the profile of the proximal self-expanding internal sealing edge (111) of the conduit is designed as a combination of all or part of: an outward radial section (121), an inward radial section (122), an horizontal linear section (123), and a vertical linear section (124), to enhance its self-collapsibility and minimize the pressure applied on the intestine wall, while maintaining its sealing efficacy.

In certain embodiments of the ostomy draining device according to any of the embodiments above, the radius of the outward and inward radial sections (121, 122) of the conduit (101) is: from 0.5 to 5mm; from 0.75 to 5mm; from 1 to 5mm; from 1.5 to 5mm; from 2 to 5mm; from 2.5 to 5mm; from 0.5 to 4.5mm; from 0.5 to 4mm; from 0.5 to 3.5mm; from 0.5 to 3mm; and from 1.5 to 3.5mm. In certain embodiments, its thickness is: from 0.2 to 2mm; from 0.3 to 2mm; from 0.4 to 2mm; from 0.5 to 2mm; from 0.6 to 2mm; from 0.7 to 2mm; from 0.8 to 2mm; from 0.9 to 2mm; from 0.2 to 1.9mm; from 0.2 to 1.8mm; from 0.2 to 1.5mm; from 0.2 to 1.2mm; from 0.3 to 1.2mm; from 0.2 to 1mm; or from 0.3 to 0.9mm. In further embodiments, its length of the radial section is: from 0.2 to 7mm; from 0.3 to 7mm; from 0.4 to 7mm; from 0.5 to 7mm; from 0.2 to 6mm; from 0.2 to 5mm; from 0.2 to 4mm; from 0.2 to 3mm; from 0.4 to 3mm; or from 0.5 to 2.5mm. The angle (127) of the horizontal linear section is 89° from to 25°, preferably 65° to 35°, the thickness of the horizontal linear section is from 0.2 to 2mm, preferably 0.3-0.9mm, and its length is from 2 to 12mm, preferably 4-9mm. The angle (127) of the vertical linear section is from 20° to 1°, preferably 7° to 2°, its thickness is from 0.2 to 2mm, preferably 0.3-0.9mm, and the length of the vertical linear section is from 2 to 12mm, preferably 6-10mm.

In certain embodiments of the ostomy draining device according to any of the embodiments above, the proximal self-expanding internal sealing edge (111) of the conduit is equipped with one or more O-rings (125,126) to improve and facilitate its self-expandability and sealing efficacy. The O-rings (125,126) may be located at one or more of the linear or curved sections of the sealing edge (111). The O-rings may be external (126) to improve sealing in case of irregular intestine profile, or internal (125), to minimize high point pressure on the intestine wall. In specific embodiments, the O-rings' radius is: from 0.2 to 1.5mm; from 0.3 to 1.5mm; from 0.4 to 1.5mm; from 0.5 to 1.5mm; from 0.2 to 1.4mm; from 0.2 to 1.3mm; from 0.2 to 1.2mm; from 0.2 to 1.1mm; from 0.2 to 1mm; from 0.3 to 1.4mm; from 0.4 to 1.3mm; from 0.4 to 1.2mm; or from 0.5 to 1mm.

In certain embodiments of the ostomy draining device of the invention, which comprises a detachable applicator (109) as described hereinabove, the applicator (109) prevents the attachment of a stoma-bag to the conduit or flange, thereby forcing the user to first insert the conduit (101) properly into the stoma, remove the applicator (109) after the conduit is properly positioned in place, and only then connect the stoma-bag. This assures that the applicator is removed before the bag connection and that it is not forgotten inside the conduit.

In certain embodiments of the ostomy draining device according to any of the embodiments above, the conduit (101) is fully or partially covered with a polymeric membrane. In specific embodiments, the polymeric membrane is inside or outside the conduit's wall. Notably, in case the bare conduit is porous, a coating membrane is required for, e.g., sealing the conduit to prevent leakage and facilitate draining of, e.g., stool, sealing the conduit to the stoma inner wall, and preventing penetration of intestinal mucosa and fluids from penetrating the conduit's surface/walls. In further specific or alternative embodiments, the self-expanding internal sealing edge (111) of the conduit is made of a polymeric membrane.

In certain embodiments of the ostomy draining device according to any of the embodiments above, the external draining edge of the conduit (101) is attached to a flexible sealing sleeve (118). In specific embodiments thereof, the conduit (101) is crimped by stretching/pulling the internal sealing edge inwards by pulling an applicator (109).

In certain embodiments of the ostomy draining device according to any of the embodiments above, the conduit's outer surface comprises sections applying lower radial force on the intestine internal wall/stoma's opening constantly or alternately to, e.g., enable temporary relief and avoid constant pressure on the intestinal wall/stoma's opening and mucosa that may cause erosion and necrosis. This enables placing the conduit inside a stoma for a prolong period of time without causing damage to the tissue (the stoma and surrounding skin) and without causing discomfort or irritation to the user.

In certain embodiments, the ostomy draining device according to any of the embodiments above further comprises a polymeric intestinal gas squirt acoustic silencer, said silencer comprising: (a) an inlet connector designed to associate with the conduit's external draining edge and/or a (polymeric) flange (106) attached to the conduit's external draining edge; (b) a main body designed to act as the silencer; and (c) a gas exhaust outlet. This allows silent release of gas from the device when installed thereby eliminating unpleasant sounds associated with release of such gases. In specific embodiments, the silencer is made-of or coated-with or further comprises a gas filter with an odor absorbance layer to obviate odor release when the gas is discharged. Accordingly, in specific embodiments, a noise acoustic silencer as defined herein is attached to the conduit's outer flange (106) to mute intestine gas squirt embarrassing noises.

In certain embodiments of the ostomy draining device according to any of the embodiments above, the stoma-bag flange connector (105) is equipped with a stoma-bag washer comprising: (a) a circular pierced sprinkler tube attached to the stoma-bag flange connector's inner circumference; (b) a flexible washing tube connected to said sprinkler tube; and (c) a flexible rubber nozzle welded to the other edge of said flexible washing tube and designed to associate with a faucet mouthpiece.

Accordingly, in specific embodiments of the baseplate (102), the flange (105) is integrated with an inner circular pierced tube (112) connected to a short flexible tube (113) with flexible rubber adapter (114). The rubber adapter (114) is used to attach the tube (113) to a common washbasin faucet and easily wash a drainable stoma-bag without risk of self or environment dirtying.

In certain embodiments, the ostomy draining device according to any of the embodiments above, further comprises a leak-alert means/detector designed to alert the patient using the device that there is a leak, and that the device needs to be replaced before serious leak and damage occur. Such leak-alert means/detector may be, e.g., chemical (e.g. pH indicator) or electro-chemical (e.g. conductivity), and the alert may be visual (e.g. color change) or audio alert, or both. In specific or alternative embodiments, the leak detector sends an alert to a remote device, such as a smartphone, that alerts the user of a leak. One such possible detector is a color-changing element/ layer that changes its color according to pH- accordingly, a stool leak, which has a low pH, would cause the layer to change its color. Another such means is an alarm that sounds an alert when identifying a leak-, e.g., by getting wet or pH change. Yet another means is an electronic alert that upon identification of a leak sends the patient an electronic alert, such as an SMS, WhatsApp or email. Accordingly, in specific embodiments, such a leak-alert means/detector is a layer or coating over the outer surface of the conduit's external draining edge with a pH or other conductivity color-changing element that is designed to change color when exposed to an intestinal liquid. In specific embodiments, the leak-alert detector is attached to the conduit adjacent to the stoma to provide an alert before a leak becomes significant.

In certain embodiments of the ostomy draining device according to any of the embodiments above, the conduit's length is of 50 to 120mm; 60 to 120mm; 70 to 120mm; 80 to 120mm; 90 to 120mm; 100 to 120mm; 50 to 110mm; 50 to 100mm; 50 to 90mm; 60 to 110mm; 70 to 100mm; or 80 to 110mm, the conduit's expanded diameter is of: 2.5-50mm; 3.5-50mm; 5-50mm; 7.5-50mm; 10-50mm; 2.5-45mm; 5-45mm; 7.5-45mm; 10-45mm; 2.5-40mm; 5-40mm; 7.5-40mm; 10-40mm; 2.5-35mm; 5-35mm; 7.5-35mm; 10-35mm; 2.5-30mm; 5-30mm; 7.5-30mm; 10-30mm; 2.5-25mm; 5-25mm; 7.5-25mm; 10-25mm; 10-20mm; 15-50mm; 20-50mm; 25-50mm; 30-50mm; or 35-50mm; wherein the conduit's length and diameter are dependent on (a) the ostomy type (colostomy / ileostomy / urostomy); and (b) the ostomate's personal dimensions. In further specific embodiments, the conduit's wall thickness is of 0.05-1.00mm.

In specific embodiments, the present invention provides an ostomy disposable device comprising three main components: (1) an ostomy sealing and draining self-expanding covered conduit (101) according to any of the embodiments above, wherein the conduit has an inner sealing flared edge and a draining connection outer edge, crimped by a flexible polymeric shrinking/crimping sleeve; (2) a body attachment baseplate (102) consisting essentially entirely of a stoma-bag adapter flange (105) with stoma belt buckle connections and body attachment baseplate sheet with high friction baseplate layer; and (3) a flexible sleeve adapter (118) to connect the conduit's flange with the baseplate (102).

In further specific embodiments, the conduit (101) is crimped prior to insertion into the stoma by the ostomate, and then inserted thereby to a position where the outer conduit's edge is adjacent to the stoma's end. Once the conduit (101) is in place, the conduit flexible polymeric shrinking/crimping sleeve that is responsible for crimping the conduit (101) is removed thereby allowing the conduit (101) to expand. This secures the conduit in place and seals the intestine's inner wall by attaching the inner flared edge to the intestine inner wall at the inner belly wall section and forming an artificial synthetic stoma mouthpiece.

Contrary to current stoma devices, the baseplate (102) according to any of the embodiments above is not glued to the ostomate's body, but is held in place by, e.g., an elastic stoma belt, while shifting and movement of the baseplate (102) is prevented thanks to the presence of a high friction sticky polymeric layer at the baseplate's surface facing the ostomate's skin. The fact that the baseplate is not glued to the ostomate's skin is advantageous over known stoma devices in that it eliminates the risk of skin irritations, allergies, rashes, itching and pealing skin. Moreover, it enables fast and easy installation / replacing of the baseplate- even when the ostomate has an irregular stoma or suffers from various skin conditions. Furthermore, the flexible sleeve adapter (118) provides the sealing between the conduit's outer edge where the intestine stool is secreted and the baseplate's flange (105) to which the stoma-bag is attached.

In certain embodiments, the ostomy draining device according to any of the embodiments above, is a one-piece ostomy device, wherein the conduit's external edge is connected by a flexible sleeve adapter (118) to a stoma-bag flange connector (105) that is pre-welded, pre-adhered, or pre-assembled to a baseplate (102).

In certain embodiments of the ostomy draining device of any of the embodiments above, the baseplate (102) is made of elastic polymer, rectangular, circular or any other geometrical shape, with a baseplate layer made of bribed sticky silicone or any other sticky high friction textile, woven or unwoven fabric to prevent the baseplate from sliding on the skin while maintaining ventilation to the skin. In specific embodiments, a rigid polymeric stoma-bag flange connector (105) is attached to the baseplate and a couple of stoma elastic belt buckles are attached to the peripheral scope of the flange (105) or the baseplate, to enable connection of a stoma belt.

In certain embodiments, the conduit (101) and the ostomy device according to any of the embodiments above are made from any suitable material. The materials used are not particularly limited and all common existing stoma appliances and colonic, esophagus, biliary or airway conduits materials may be used. In addition, other conduit types, designs and fabrication processes may be employed/used. For example, a Self-Expendable-Metallic-Conduit made of nitinol metal, biodegradable polymer or alloy; balloon-expanded conduit; helical, wire-weaved, laser cut, molded or 3D-printed conduit and alike.

In certain embodiments, the ostomy device according to any of the embodiments above overcomes most of the prior art problems and allows for a simplified technique for self-applying a stool collecting device to the stoma, the device provides at least the following advantages:
(a) leak-proof device with excellent seal of stoma-bag to the stoma regardless of stoma or skin conditions, avoiding all harmful effects of stool leakage to skin and personal quality of life;
(b) reduce anxiety and social isolation;
(c) no long-term chronic hermetic attachment of baseplate to skin allowing access to stoma and peristomal skin without removing the stoma device. No adhesive, sealant sealing accessories or any skin protective additives required;
(d) long-term continuous conduiting significantly reduces the risk for stoma blockage due to stenosis or retraction;
(e) avoiding psychological effects of odor and gas squirt noise;
(f) low frequency baseplate changes - up to once a month; and
(g) simple and fast baseplate change procedure.

Unless otherwise indicated, all numbers used in this specification are to be understood as being modified in all instances by the term "about". Accordingly, unless indicated to the contrary, the numerical parameters set forth in this specification are approximations that may vary by up to plus or minus 10% depending upon the desired properties to be obtained by the present invention.

Reference is now made to the accompanying Drawings, which are aimed at illustrating the invention and are by no way limiting the claimed scope.

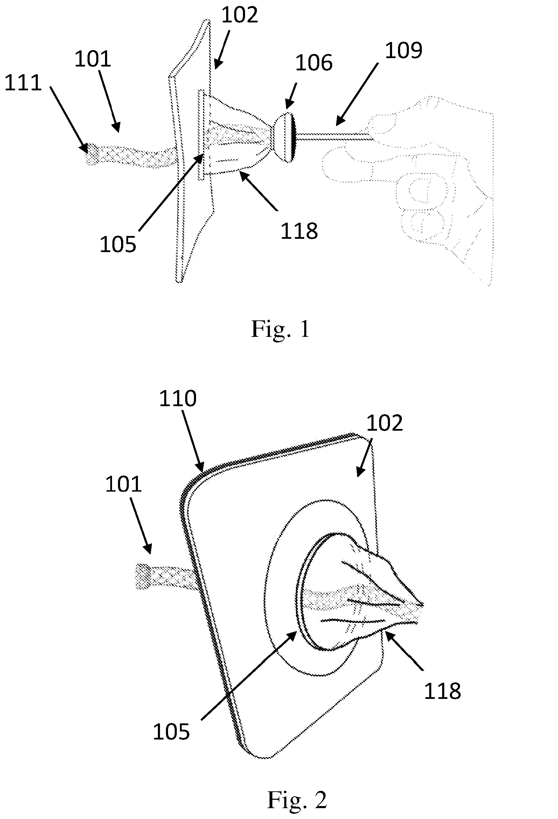

**Fig. 1** illustrates one possible configuration of three-pieces ostomy draining device of the invention, showing the conduit (101) and its proximal self-expanding internal sealing edge (111), a baseplate (102), and a flexible sealing sleeve (118). The flexible sealing sleeve is attached with the conduit's distal edge by a flange (106) and attached to the baseplate's flange (105), providing hermetic sealing between the conduit and the baseplate. This configuration guaranty that the intestine's secretion flows from the intestine directly to a stoma-bag attached to the baseplate's flange (105) with no intact with the peristomal skin while redundant the need for adhesion. As illustrated, the conduit (101) is inserted into the stoma using an applicator (109) to support and overcome undesired collapse. In the conduit illustrated in Fig. 1, the sleeve has stent capabilities, meaning that it is designed to be widen after insertion into the stoma for both anchoring the device inside the stoma and to facilitate sealing, by applying radial force/pressure of the conduit's outer walls against the stoma's inner walls.

**Fig. 2** is another possible configuration of one-pieces ostomy draining device of the invention, where the flexible sealing sleeve (118) is welded or associated with the conduit's distal edge (101) and the baseplate's flange (105). This configuration is more robust and lower cost but less adjustable and configurable.

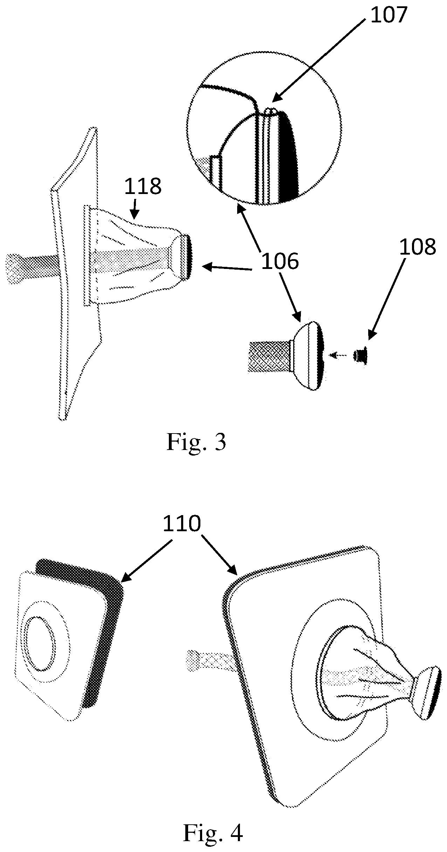

**Fig. 3** further illustrates in detail the connection of the flexible sealing sleeve (118) to the distal edge of the conduit (101) in a three-pieces device configuration, as illustrated in **Fig. 1****,** and the use of a plug (108) at the connection flange (106). As illustrated, the plug (108) is detachable from the flange (108), but it is to be understood that it may be connected or constructed as an integral part of the flange (108) designed to be automatically opened once a stoma-bag is connected thereto, and automatically closed once the stoma-bag is disconnected/removed therefrom.

**Fig. 4** illustrates the gas absorbance filter (110) located at the inner layer of the baseplate (102) that is designed to be in contact with the ostomate's skin and absorb odors and prevent bad smells. Two designs are exemplified: with and without a sleeve.

**Fig. 5** illustrates various possible components of the ostomy draining device of the invention designed to enable easy and sanitary washing of the stoma-bag, e.g. after stoma-bag draining. As seen, a circular pierced tubular ring (112), designed as a sprinkler, is connected to the baseplate's flange (105), enabling rinsing the stoma-bag connected to the baseplate's flange (105). The flexible tube (113) is designed with a flexible cup edge (114) that may be connected to a common washbasin faucet thereby enabling easily washing the stoma-bag internal after draining the stool.

**Fig. 6** provides a 3-Dimentional cross-view of a three-pieces conduit configuration (baseplate is not shown). Such a conduit can be a stand-alone ostomy draining device, in which the cap with the flange constitutes the baseplate to which the stoma-bag is connected. As seen, the conduit has a proximal self-expanding internal sealing edge (111) with a sleeve

(119) designed to extend outwards through the stoma's opening, wherein the conduit is secured in place by placing an external membrane/cap (118) onto the distal external draining edge. The cap (118) is maintained onto the sleeve thanks to the interaction between dedicated protrusions and rims (116). These protrusions and rims (116) further enable to adjust the length of the sleeve, thereby enabling adjustment of the conduit to different stoma thicknesses. **Fig. 6** further illustrates the possibility of using reinforcement rigid rings (115) near or at the expanding internal sealing edge (111) to prevent its axial flipping as well as axial collapsing of the internal sealing edge (111) during insertion into the stoma and while residing therein.

**Fig. 6** further illustrates another possibility to secure the sleeve (119) in place using a self-expandable support stent (120) within the connecting sleeve (119) is connected to the baseplate (105) (not shown in this drawing) *via* a flexible sealing sleeve (118) as shown in **Fig. 1****,** instead of an elastomeric membrane (118).

**Figs. 7A-7D** illustrate various designs and combinations of curved and linear sections of the self-expanding internal sealing edge (111), inward or outward radial section (121,122) with a different angle thereof (127), wider opening, thicker walls, longer / shorter vertical linear section (124), with or without O-ring(s) (125,126); all designed to enhance its self-collapsibility and minimize the pressure applied on the intestine wall, while maintaining its sealing efficacy.

**Fig. 8** illustrates how the device of the invention is positioned within a stoma, such that the self-expanding internal sealing edge (111) is positioned inside the patient's intestine and the elastomeric external cap (118) is positioned outside the stoma, applying axial force on the abdomen's external wall (130) to tighten the internal sealing edge (111) to the abdomen's internal wall (129) and prevent leakage of fluids and allows passage of such fluids only via the conduit's hollow tube (119) (see dotted arrows).

**Figs. 9A-9D** illustrate the assembly of a three-pieces device. The conduit's sleeve (119) and the external membrane (118) **(****Fig. 9A****)** are assembled together and length is adjusted by the reems (116) **(****Fig. 9B****).** Finally, the baseplate (102) **(****Fig. 9C****)** is attached to the external membrane (118) **(****Fig. 9D****).**

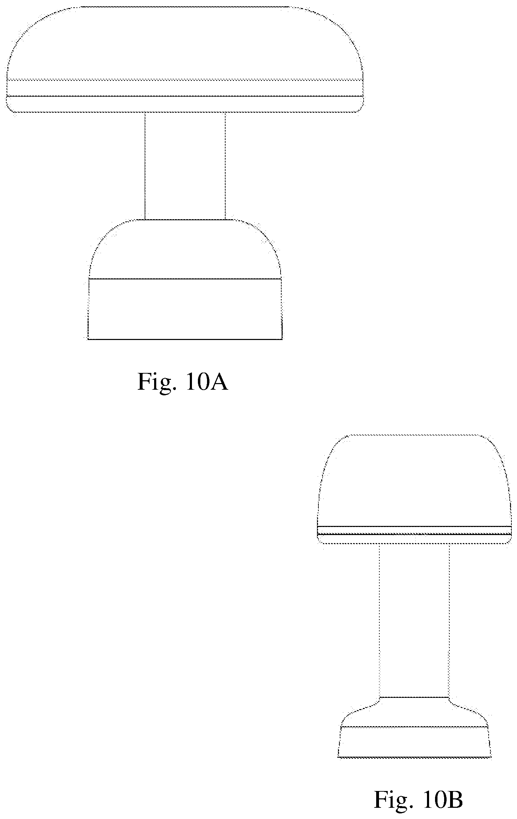

**Figs. 10A-10B** illustrate two optional designs of the external membrane (118). **Fig. 10A** illustrates a large diameter and short external membrane that provides a weaker axial force and shorter linear compensation and therefor require more robust internal sealing edge. **Fig. 10A** illustrates a small diameter and high external membrane that provides a stronger axial force and longer linear compensation and therefor enables more gentle internal sealing edge.

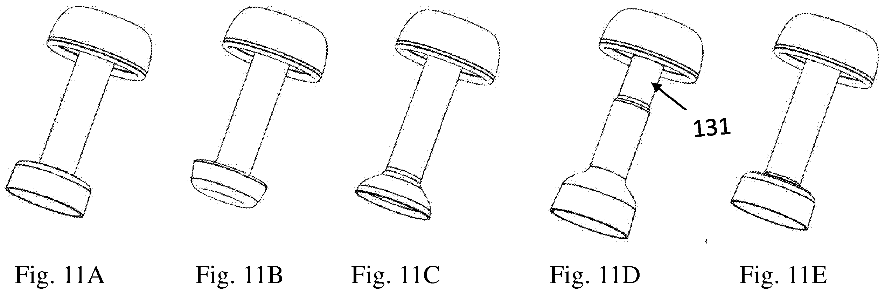

**Figs. 11A-11E** are illustrations of different possible designs and combinations of a two-pieces device designs according to the invention. The conduit is integrated with the external membrane to a one unit with a pre-defined, non-adjustable length, and the baseplate (not shown) is separate. Each conduit is designed according to need, e.g., with axially collapsible sleeve (131 in **Fig. 11D****),** inverted internal sealing edge **(****Fig. 11B****),** with **(****Fig. 11C****)** or without **(****Fig. 11D****)** O-ring(s), with **(****Fig. 11A****)** or without **(****Fig. 11C****)** vertical linear section, etc.

**Figs. 12A-12D** are illustrations of a two-pieces device as in **Fig. 11****,** with longer **(****Figs. 12A-12C****)** or shorter **(****Fig. 12D****)** sleeve, enforcement ring **(****Figs. 12B & 12D****)** and specific internal sealing edge (111) shapes, etc.

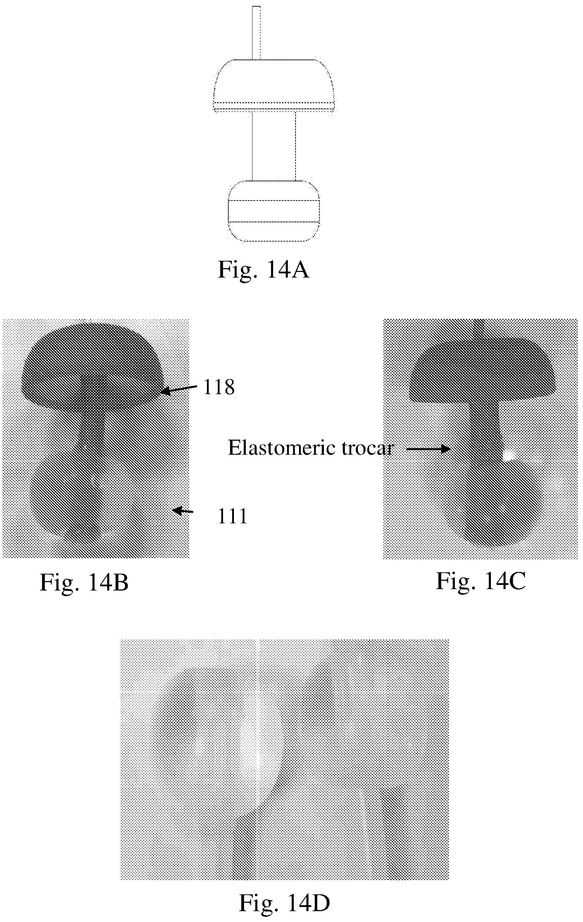

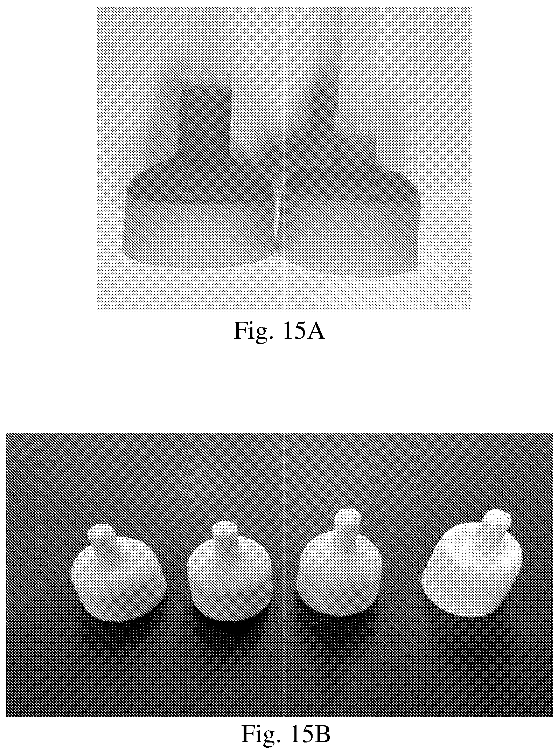

**Figs. 14A-14D** are pictures of different possible designs and combinations of the self-expanding internal sealing edge of the conduit, showing a balloon-based internal sealing edge (111) made of silicone **(****Figs. 14B-14C****)** or polyurethane **(****Fig. 14D****)** and the balloon sealing edge is normal **(****Fig. 14B****)** or inverted **(****Fig. 14C****),** and **Figs. 15A****-15B** are pictures of different possible designs and combinations of the self-expanding polymeric internal sealing edge (111) made of silicone **(****Fig. 15A****)** or polyurethane **(****Fig. 15B****).**

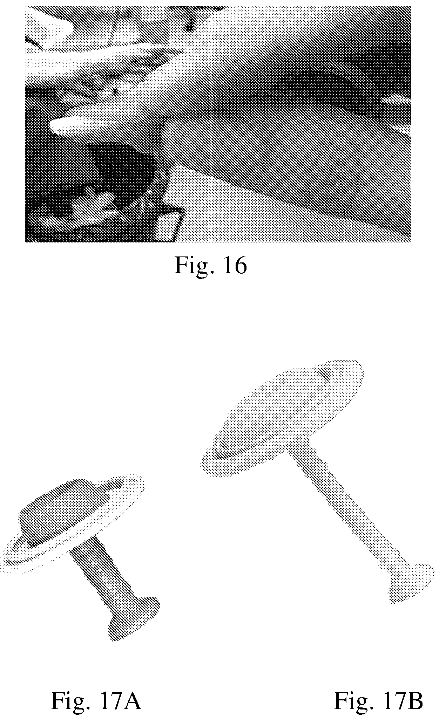

**Fig. 16** illustrates how to fold the self-expanding internal sealing edge (111) of the conduit on the applicator prior to its insertion into the stoma. As illustrated, the folding is carried out manually to ease the initialization of the conduit's self-collapsing. However, the folding may be done by the applicator as well. In case of a conduit that is not self-collapsing, the crimping can be done in any other technique and the conduit may even arrive/stored in a crimped form, ready for insertion as explained above.

**Figs. 17A-17B** are pictures of conduits of the invention in different dimensions, used during *in-vivo* experiments on pigs. **Fig. 17A** is a three-pieces device as illustrated in **Fig. 9****;** and **Fig. 17B** is a two-pieces device where the baseplate is made of silicone and integrated with the external membrane and the sleeve is connected to the membrane by the rims.

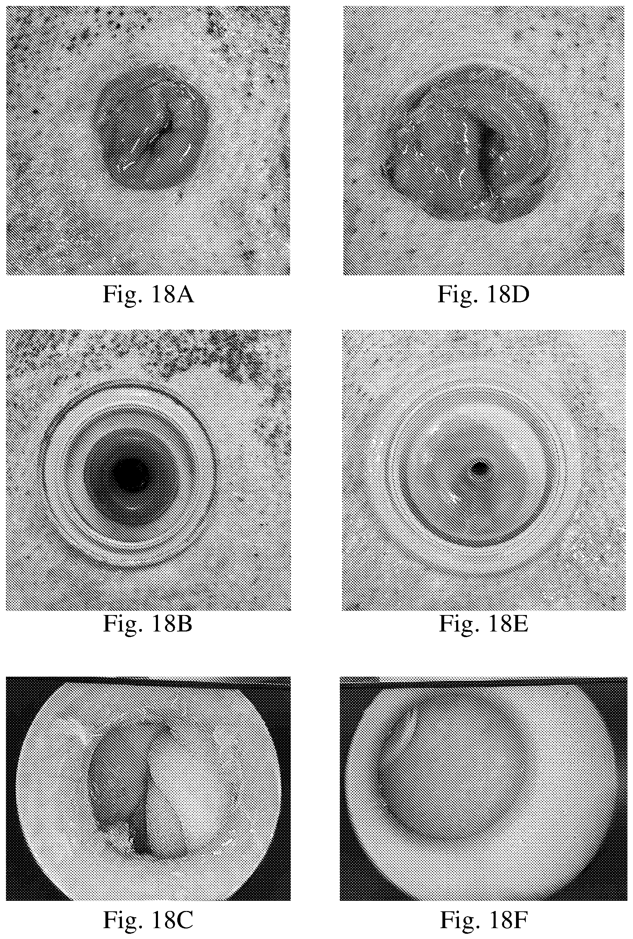

**Figs. 18A-18F** are pictures taken during *in-vivo* experiments on pigs using conduits of the invention. **Figs. 18A & 18D** show the stoma after 1 and 4 weeks respectively; **Figs. 18B & 18E** show how three-pieces conduit and two-pieces conduit used in the 1^{st} and 4^{th} weeks (respectively) are placed within the stoma and covers it; and **Figs. 18C & 18F****,** are endoscopic images showing the conduit placed in the stoma and that the tissues in the stoma and surrounding it are healthy, and that there is no sign of infection or leakage.

While various embodiments have been described above, it should be understood that they have been presented by way of example only, and not limitation. Where methods described above indicate certain events occurring in certain order, the ordering of certain events may be modified. Additionally, certain of the events may be performed concurrently in a parallel process when possible, as well as performed sequentially as described above.

### EXAMPLES

### Example 1 - Ex-vivo Trial Summary and Concept Decision

The purpose of this trial is to evaluate two families of inner-sealing solutions: self-expanding Cup-Shaped Elastomer sealing **(****Figs. 15A & 15B****);** and Balloon-based inner sealing **(****Figs. 14C, 14D** **& 14E).** The evaluation is done over an Ileostomy *ex-vivo* model simulator to decide which of the two solutions is preferable.

We used swine Ileostomy model and performed a comparison set of tests for evaluation. Five different elastomer-based models and 4 balloon-based models were tested. All models were inserted and tested based on the following protocol:
1) Introduction of the model into the Ileostomy simulator;
2) Evaluate ease of device introduction into the stoma;
3) Verify the elastomeric structure is open / balloon is inflated to a predefined pressure (inner sealing device should move freely (<90gr) within the ileum colon);
4) Apply incremental water pressure from 5cm/H₂O up to 30cm/H₂O (based on literature review):
   a. Measure priming axial force required to achieve water seal at every water pressure; and
   b. Verify no leakage while radial movement of the protruding Trocar at various water pressure; and
5) Measure extraction force of the device from the stoma at atmospheric pressure.

### Results:

The Ileum colon inner diameter: at atmospheric pressure: 23mm; and at 10cm/H₂O: 24mm. Two model families were tested: Family I- Elastomer; and Family II- Balloon.

From Elastomer family: 3 types of Thermoforming TPU cones and 2 types of silicon cones were used (all over HDPE ML tube) - see illustrated in **Figs. 15A & 15B****.**

**Table 1 - Elastomer: TPU A**

| | Evaluate | Pressure [cm/H₂O] | | | | | | | Comments |
|---|---|---|---|---|---|---|---|---|---|
| | 1....5 | 0 | 5 | 10 | 15 | 20 | 25 | 30 | |
| | Easy...Hard | | | | | | | | |
| Introduction | 1 | NA | NA | NA | NA | NA | NA | NA | Introduction using external tube |
| Extraction | 3 | NA | NA | NA | NA | NA | NA | NA | Xgr. Minimal colon everted during extraction |
| Seal w/ Priming only | NA | √ | √ | √ | √ | √ | √ | √ | Xgr to Ygr priming. |
| Seal w/ Radial movements | NA | √ | √ | √ | √ | √ | √ | √ | Xgr to Ygr priming. Stable and good sealing |

**Table 2 - Silicon D**

| | Evaluate | Pressure [cm/H₂O] | | | | | | | Comments |
|---|---|---|---|---|---|---|---|---|---|
| | 1....5 | 0 | 5 | 10 | 15 | 20 | 25 | 30 | |
| | Easy...Hard | | | | | | | | |
| Introduction | 1 | NA | NA | NA | NA | NA | NA | NA | Introduction using external tube |
| Extraction | 3 | NA | NA | NA | NA | NA | NA | NA | Xgr. Minimal colon everted during extraction |
| Seal w/ Priming only | NA | √ | √ | √ | √ | √ | √ | √ | Xgr to Ygr priming. |
| Seal w/ Radial movements | NA | √ | √ | √ | √ | √ | √ | √ | Xgr to Ygr priming. Stable and good sealing |

### Conclusions:

Both model families had a good and reliable sealing with different priming force. Balloon family is more sensitive to internal abdominal surface and to balloon symmetry and therefore tends to require more priming force to deal with inconsistencies. Elastomeric family on the other hand deals better with variety of inconsistency, has the freedom for design improvement in sealing surfaces. Balloon family is more complicated to manufacture and build.

To summarize, it seems that the Silicon Elastomer family is preferred.

### Example 2 - Ex-vivo Study

Six silicon-elastomer models were tested **(****Figs. 11A-11E****),** all having a drainage tube (connecting the inner membrane to the outer membrane) suitable for abdominal tissue of 4-5 cm thickness having different seal shapes and Shore A.:

### Conclusions:

The length of the drainage tube should be manufactured according to the thickness of the tissue and/or the external membrane should be movable along the drainage tube to enable adjustment to the tissue's thickness.

The collapsible drainage tube has no effect on the sealing capabilities, while reducing the effect of external pressure, e.g. during insertion or during exercise and effort.

The device can be inserted into the stoma, e.g., by using an internal support, such as an internal dilator, while avoiding flipping of the skirt.

The experiment results were used to define the best designs to achieve good sealing, usability and safety.

### Example 3 - In-vivo Study

This experiment is designed for the evaluation and testing of the efficacy, performance, and safety of a novel ileostomy stoma device [SilTop^{™} of StomTop Ltd.], which addresses the need for a leak-proof, adhesive-free stoma device for the management of ileostomy.

Specifically, this experiment is aimed at evaluating the safety and ease of insertion and removal of the various device's prototypes, as well as the sealing efficacy of various devices prototypes at various pressures ranges at rest. This was done by testing various device designs.

End points: Leakage test on an anesthetized animal for 30 min.

Three types of SilTopTM configurations were tested for efficacy: SilTopTM #13, #15, #18, differ by seal diameter and angle.

Each SilTop configuration was inserted using the Punch-Down Fold technique, in which the tip of the device is folded before insertion into the stoma, such that upon insertion therein it self-collapses and expands to its original shape upon positioned. See illustration in **Fig. 16****.**

The test was initiated with a quality measure of device insertion and quantitative measure of pull force extraction for each device configuration. Further measurements included sealing tests at rest over variable pressures ranging from 5cm/H₂O to 30cm/H₂O followed by a sealing test during radial manipulation of the devices.

### Results:

**Table 3 - Usability**

| **Test #** | **Test Name** | **Test Parameter** | **Test Result** | **Notes** |
|---|---|---|---|---|
| **Config. #18** | Device Insertion | • Ease of insertion evaluation | • Very easy to insert (5) | • Punch-Down fold technique |
| | | • Internal membrane deployment efficacy | • Membrane self-deployed | • Endoscope image for membrane deployment |
| | | | • No tissue damage | |
| | | • Tissue damage | | • Endoscope image for tissue damage |
| | Device Removal | • Ease of removal evaluation | • Very easy to remove (5) | • Force gauge |
| | | | | • Endoscope image for tissue damage |
| | | • Removal force | • 190 gr for initial movement and pick pull force | |
| | | • Tissue damage | | |
| | | | • No tissue damage | |
| **Config. #15** | Device Insertion | • Ease of insertion evaluation | • Very easy to insert (5) | • Punch-Down fold technique |
| | | • Internal membrane deployment efficacy | • Membrane self-deployed | • Endoscope image for membrane deployment |
| | | | • No tissue damage | |
| | | • Tissue damage | | • Endoscope image for tissue damage |
| | Device Removal | • Ease of removal evaluation | • Very easy to remove (5) | • Force gauge |
| | | | | • Endoscope image for tissue damage |
| | | • Removal force | • 170 gr for initial movement and up to 220 gr pick pull force | |
| | | • Tissue damage | | |
| | | | • No tissue damage | |
| **Config. #13** | Device Insertion | • Ease of insertion evaluation | 1. Easy to insert (4) | 1. Punch-Down fold technique |
| | | | 2. Easy but a bit harder (3) | |
| | | • Internal membrane deployment efficacy | | 2. Rotational technique |
| | | | • Membrane self-deployed | |
| | | | | • Endoscope image for membrane deployment |
| | | • Tissue damage | • No tissue damage | |
| | | | | • Endoscope image for tissue damage |
| | Device Removal | • Ease of removal evaluation | • Very easy to remove (5) | • Force gauge |
| | | • Removal force | • 270 gr for initial movement and up to 350gr pick pull force | Endoscope image for tissue damage. |
| | | • Tissue damage | • Slight tissue damage | Damage most probably following many insertions/removals and not specifically because of Device #13 |
| | | | | |

Two insertion techniques were examined: the "Punch-Down Push" and "Rotational Push". The first was easier and therefore used in most cases.

**Table 4 - Sealing at rest**

| **Test #** | **Test Name** | **Test Parameter** | **Test result** | **Notes** |
|---|---|---|---|---|
| **Config. #15** | @5cm/H₂O | Sealing for 5 min | No leaks observed | Used both mm scale and digital pressure sensor |
| | @10cm/H₂O | Sealing for 7 min | No leaks observed | Used both mm scale and digital pressure sensor |
| | @20cm/H₂O | Sealing for 5 min | No leaks observed | Used both mm scale and digital pressure sensor |
| | @30cm/H₂O | Sealing for 5 min | No leaks observed | Used both mm scale and digital pressure sensor |
| **Config. #18** | @5cm/H₂O | Sealing for 5 min | No leaks observed | Used both mm scale and digital pressure sensor |
| | @10cm/H₂O | Sealing for 5 min | No leaks observed | Used both mm scale and digital pressure sensor |
| | @20cm/H₂O | Sealing for 5 min | No leaks observed | Used both mm scale and digital pressure sensor |
| | @30cm/H₂O | Sealing for 5 min | No leaks observed | Used both mm scale and digital pressure sensor |
| **Config. #13** | @5cm/H₂O | Sealing for 5 min | No leaks observed | Used both mm scale and digital pressure sensor |
| | @30cm/H₂O | Sealing for 5 min | No leaks observed | Used both mm scale and digital pressure sensor |

### Conclusions:

### 2^{nd} study - in-vivo efficacy trial

After successful completion of the first animal efficacy test with three configurations, the main outcome is that the concept of elastomeric internal membrane functions well.

Each of the three configurations proved to function very well and seals at all ranges of pressure. The insertion of config. #18 was easier and probably safer since it fits inside the ileum freely while still providing an excellent sealing.

During the manipulation test an axial force (inward) was applied resulting some temporary leak. Accordingly, in certain embodiments, the conduit of the device further comprises a collapsible ridge thereover, to prevent undesired leakage due to axial force applied thereon.

While the invention has been shown and described with respect to particular embodiments thereof, those embodiments are for the purpose of illustration rather than limitation, and other variations and modifications of the specific embodiments herein described will be apparent to those skilled in the art, all within the intended scope of the invention. Accordingly, the invention is not to be limited in scope and effect to the specific embodiments herein described, nor in any other way that is inconsistent with the extent to which the progress in the art has been advanced by the invention.

## Claims

1. An ostomy draining conduit (101) comprising a proximal self-expanding internal sealing edge (111) configured to be inserted into a stoma and seal the passage between the conduit's exterior wall and the stoma's interior wall; and a distal external draining edge, such that when inserted into the stoma it allows passage of liquid and stool while preventing passage between the conduit's exterior wall and the stoma's interior wall, **characterized in that**: (i) the proximal self-expanding internal sealing edge's (111) diameter is smaller than an intestine's diameter when the conduit resides within the stoma, and (ii) the proximal self-expanding internal sealing edge (111) is self-collapsible, such that the mere pressure applied on the conduit (101) during insertion into the stoma is sufficient for it to collapse inwardly to allow its insertion into the stoma, and return to its original shape once inserted into the stoma.

2. The ostomy draining conduit (101) of claim 1 further comprising a hollow connecting sleeve (119) connecting said proximal self-expanding internal sealing edge (111) and said distal external draining edge, wherein the sleeve's length is longer than the abdomen's thickness, wherein the length of said ostomy draining conduit (101) is optionally adjustable.

3. The ostomy draining conduit (101) of claim 2, further comprising: an elastic sealing external membrane (118) mounted onto said connecting sleeve (119), wherein movement of said external membrane (118) along the sleeve (119) towards the internal sealing edge (111) creates a sealing axial force to affix said conduit (101) in place, and wherein said external membrane (118) is anchored along the sleeve (119) by dedicated protrusions and rims (116).

4. The ostomy draining conduit (101) of claim 2, wherein said sleeve (119) comprises a region that is self-collapsible, thereby preventing unintentional damage to the stoma's walls during insertion of the conduit into the stoma.

5. An ostomy draining device comprising the ostomy draining conduit (101) of any one of claims 1-4.

6. The ostomy draining device of claim 5, when dependent on claims 1-3, wherein said conduit (101) further comprises a hollow sleeve (119) connecting said proximal self-expanding internal sealing edge (111) and said distal external draining edge, wherein the sleeve's length is longer than the abdomen's thickness, wherein said sleeve (119) comprises a region that is self-collapsible, thereby preventing unintentional damage to the stoma's walls during insertion of the conduit into the stoma, and wherein the length of the ostomy draining conduit (101) is adjustable.

7. The ostomy draining device of claim 5, further comprising:
(a) a body attachment baseplate (102) for attaching said ostomy device to an ostomate, said baseplate (102) comprising a stoma-bag adapter flange (105) without an external stoma wall sealant, designed to surround the stoma's external wall without direct contact therewith, wherein said baseplate (102) is adhesive-free or non-adhesive, wherein said baseplate (102) optionally: (i) comprises a gas filter (110) with an odor absorbance layer; or (ii) is flippable to enable monitoring and management of peristomal skin under the baseplate; and
(b) an elastic sealing external membrane (118) mounted onto said connecting sleeve (119), wherein movement of said external membrane (118) along the sleeve (119) towards the expanding internal sealing edge (111) creates a sealing axial force to affix said conduit (101) in place, wherein said external membrane (118) is anchored along the sleeve (119) by dedicated protrusions rims (116).

8. The ostomy draining device of claim 7, further comprising a flexible sealing sleeve adaptor (118) integrated with said baseplate (102), such that said external draining edge of said conduit (101) enables sealed fluid connection between said conduit (101) and a stoma-bag that is connected to said stoma-bag adapter flange (105).

9. The ostomy draining device of claim 5, wherein the external draining edge of said conduit (101) comprises a detachable connection flange (106), and is optionally equipped with a capping plug (108), wherein a flexible sealing sleeve (118) is:
- attached to said detachable connection flange (106) by a clamp or an elastic ring (107); and
- made of a hydrophobic membrane filter permeable to intestine gas and impermeable to intestine liquid.

10. The ostomy draining device of claim 5, wherein said conduit (101) further comprises: (i) a self-expandable support stent (120) located therein; or (ii) one or more reinforcement rigid rings (115) configured to prevent axial collapsing and flipping of the expanding internal sealing edge (111) during insertion thereof into the stoma.

11. The ostomy draining device of claim 5, further comprising a detachable applicator (109) for inserting the conduit (101) into the stoma, wherein said detachable applicator (109) is self-collapsible when an axial force higher than 5-10N is applied, thereby preventing unintentional erosion or perforation of the intestine during insertion of the conduit into the stoma.

12. The ostomy draining device of claim 5, wherein the conduit's outer surface comprises sections configured to apply lower radial force on the intestine internal wall constantly or alternately.

13. The ostomy draining device of claim 5, wherein the stoma-bag flange connector (105) is equipped with a stoma-bag washer comprising:
a. a circular pierced sprinkler tube attached to the stoma-bag flange connector's inner circumference;
b. a flexible washing tube connected to said sprinkler tube; and
c. a flexible rubber nozzle welded to the other edge of said flexible washing tube and configured to associate with a faucet mouthpiece.

## Patentansprüche

1. Ostomie-Drainageleitung (101), aufweisend eine proximale selbstexpandierende innere Dichtkante (111), die dazu ausgebildet ist, in ein Stoma eingesetzt zu werden und den Durchgang zwischen der Außenwand der Leitung und der Innenwand des Stomas abzudichten; und eine distale äußere Drainagekante, sodass, wenn in das Stoma eingesetzt, diese den Durchgang von Flüssigkeit und Stuhl ermöglicht, während Durchgang zwischen der Außenwand der Leitung und der Innenwand des Stomas verhindert wird, **dadurch gekennzeichnet, dass**: (i) ein Durchmesser der proximalen selbstexpandierenden inneren Dichtkante (111) kleiner als ein Durchmesser eines Darms ist, wenn sich die Leitung innerhalb des Stomas befindet, und (ii) die proximale selbstexpandierende innere Dichtkante (111) selbstzusammenklappbar ist, sodass der bloße Druck, der beim Einsetzen in das Stoma auf die Leitung (101) ausgeübt wird, ausreicht, um diese nach innen zusammenklappen zu lassen, um ihr Einsetzen in das Stoma zu ermöglichen, und nach dem Einsetzen in das Stoma in ihre ursprüngliche Form zurückkehrt.

2. Ostomie-Drainageleitung (101) nach Anspruch 1, ferner aufweisend eine hohle Verbindungshülse (119), die die proximale selbstexpandierende innere Dichtkante (111) und die distale äußere Drainagekante verbindet, wobei die Länge der Hülse länger als die Dicke des Abdomens ist, wobei die Länge der Ostomie-Drainageleitung (101) wahlweise verstellbar ist.

3. Ostomie-Drainageleitung (101) nach Anspruch 2, ferner aufweisend: eine elastische Außendichtmembran (118), die auf der Verbindungshülse (119) montiert ist, wobei Bewegung der äußeren Membran (118) entlang der Hülse (119) in Richtung der inneren Dichtkante (111) eine axiale Dichtkraft erzeugt, um die Leitung (101) an Ort und Stelle zu befestigen, und wobei die äußere Membran (118) entlang der Hülse (119) durch spezielle Vorsprünge und Ränder (116) verankert ist.

4. Ostomie-Drainageleitung (101) nach Anspruch 2, wobei die Hülse (119) einen Bereich aufweist, der selbstzusammenklappbar ist, wodurch beim Einsetzen der Leitung in das Stoma unbeabsichtigter Schaden an den Wänden des Stomas verhindert wird.

5. Ostomie-Drainagevorrichtung, aufweisend die Drainageleitung (101) nach einem der Ansprüche 1-4.

6. Ostomie-Drainagevorrichtung nach Anspruch 5, wenn abhängig von Ansprüchen 1-3, wobei die Leitung (101) ferner eine hohle Hülse (119) aufweist, die die proximale selbstexpandierende innere Dichtkante (111) und die distale äußere Drainagekante verbindet, wobei die Länge der Hülse länger als die Dicke des Abdomens ist, wobei die Hülse (119) einen Bereich aufweist, der selbstzusammenklappbar ist, wodurch beim Einsetzen der Leitung in das Stoma unbeabsichtigter Schaden an den Wänden des Stomas verhindert wird, und wobei die Länge der Ostomie-Drainageleitung (101) verstellbar ist.

7. Ostomie-Drainagevorrichtung nach Anspruch 5, ferner aufweisend:
(a) eine Körperbefestigungsgrundplatte (102) zum Befestigen der Ostomie-Vorrichtung an einem Stomaträger, wobei die Grundplatte (102) einen Stomabeutel-Adapterflansch (105) ohne ein äußeres Stomawand-Dichtungsmittel aufweist, der dazu ausgestaltet ist, die äußere Wand des Stomas, ohne direkten Kontakt damit, zu umgeben, wobei die Grundplatte (102) klebstofffrei oder nicht-adhäsiv ist, wobei die Grundplatte (102) wahlweise: (i) einen Gasfilter (110) mit einer Geruchsabsorptionsschicht aufweist; oder (ii) umlegbar ist, um Überwachung und Behandlung der peristomalen Haut unter der Grundplatte zu ermöglichen; und
(b) eine elastische Außendichtmembran (118), die auf der Verbindungshülse (119) montiert ist, wobei Bewegung der äußeren Membran (118) entlang der Hülse (119) in Richtung der selbstexpandierenden inneren Dichtkante (111) eine axiale Dichtkraft erzeugt, um die Leitung (101) an Ort und Stelle zu befestigen, wobei die äußere Membran (118) entlang der Hülse (119) durch spezielle Vorsprünge und Ränder (116) verankert ist.

8. Ostomie-Drainagevorrichtung nach Anspruch 7, ferner aufweisend einen flexiblen Dichtungshülsenadapter (118), der in die Grundplatte (102) integriert ist, so dass die äußere Drainagekante der Leitung (101) eine dichte Fluidverbindung zwischen der Leitung (101) und einem Stomabeutel, der mit dem Stomabeutel-Adapterflansch (105) verbunden ist, ermöglicht.

9. Ostomie-Drainagevorrichtung nach Anspruch 5, wobei die äußere Drainagekante der Leitung (101) einen lösbaren Verbindungsflansch (106) aufweist und wahlweise mit einem Verschlussstopfen (108) versehen ist, wobei eine flexible Dichtungshülse (118):
- an dem lösbaren Verbindungsflansch (106) durch eine Klemme oder einen elastischen Ring (107) befestigt ist; und
- aus einem hydrophoben Membranfilter hergestellt ist, der für Darmgas durchlässig und für Darmflüssigkeit undurchlässig ist.

10. Ostomie-Drainagevorrichtung nach Anspruch 5, wobei die Leitung (101) ferner aufweist:
(i) einen selbstexpandierbaren Stützstent (120), der darin angeordnet ist; oder (ii) einen oder mehrere Verstärkungsringe (115), die dazu ausgebildet sind, axiales Zusammenklappen und Umlegen der selbstexpandierenden inneren Dichtkante (111) beim Einsetzen dieser in das Stoma zu verhindern.

11. Ostomie-Drainagevorrichtung nach Anspruch 5, ferner aufweisend einen lösbaren Applikator (109) zum Einsetzen der Leitung (101) in das Stoma, wobei der lösbare Applikator (109) selbstzusammenklappbar ist, wenn eine axiale Kraft größer als 5-10 N aufgebracht wird, wodurch beim Einsetzen der Leitung in die Darmöffnung unbeabsichtigte Erosion oder Perforation des Darms verhindert wird.

12. Ostomie-Drainagevorrichtung nach Anspruch 5, wobei die äußere Oberfläche der Leitung Abschnitte aufweist, die dazu ausgebildet sind, eine geringere radiale Kraft konstant oder abwechselnd auf die innere Darmwand auszuüben.

13. Ostomie-Drainagevorrichtung nach Anspruch 5, wobei der Stomabeutel-Flanschverbinder (105) mit einer Stomabeutel-Wäscher ausgestattet ist, aufweisend:
a. ein kreisförmiges durchlochtes Sprinklerrohr, das an dem inneren Umfang des Stomabeutel-Flanschverbinders angebracht ist;
b. einen flexiblen Waschschlauch, der mit dem Sprinklerrohr verbunden ist; und
c. eine flexible Gummidüse, die an dem anderen Ende des flexiblen Waschschlauchs angeschweißt ist und dazu ausgebildet ist, mit einem Wasserhahnmündstück verbunden zu werden.

## Revendications

1. Conduit de drainage de stomie (101) comprenant un bord de scellage interne proximal auto-expansible (111) configuré pour être inséré dans une stomie et sceller le passage entre la paroi extérieure du conduit et la paroi intérieure de la stomie, et un bord de drainage externe distal, de telle sorte que lorsqu'il est inséré dans la stomie, il permet un passage de liquide et de matières fécales tout en empêchant un passage entre la paroi extérieure du conduit et la paroi intérieure de la stomie, **caractérisé en ce que** : (i) le diamètre du bord de scellage interne proximal auto-expansible (111) est inférieur au diamètre d'un intestin lorsque le conduit réside dans la stomie, et (ii) le bord de scellage interne proximal auto-expansible (111) est auto-aplatissable, de telle sorte que la simple pression appliquée sur le conduit (101) pendant l'insertion dans la stomie est suffisante pour qu'il s'aplatisse vers l'intérieur pour permettre son insertion dans la stomie, et retourne à sa forme d'origine une fois inséré dans la stomie.

2. Conduit de drainage de stomie (101) selon la revendication 1, comprenant en outre un manchon de raccordement creux (119) reliant ledit bord de scellage interne proximal auto-expansible (111) et ledit bord de drainage externe distal, dans lequel la longueur du manchon est plus longue que l'épaisseur de l'abdomen, dans lequel la longueur dudit conduit de drainage de stomie (101) est facultativement réglable.

3. Conduit de drainage de stomie (101) selon la revendication 2, comprenant en outre : une membrane de scellage externe élastique (118) montée sur ledit manchon de raccordement (119), dans lequel un mouvement de ladite membrane externe (118) le long du manchon (119) vers le bord de scellage interne (111) crée une force axiale de scellage pour fixer ledit conduit (101) en place, et dans lequel ladite membrane externe (118) est ancrée le long du manchon (119) par des saillies et des rebords (116) dédiés.

4. Conduit de drainage de stomie (101) selon la revendication 2, dans lequel ledit manchon (119) comprend une zone qui est auto-aplatissable, en empêchant ainsi un endommagement involontaire des parois de la stomie pendant l'insertion du conduit dans la stomie.

5. Dispositif de drainage de stomie comprenant le conduit de drainage de stomie (101) selon l'une quelconque des revendications 1 à 4.

6. Dispositif de drainage de stomie selon la revendication 5, lorsque dépendante des revendications 1 à 3, dans lequel ledit conduit (101) comprend en outre
un manchon creux (119) reliant ledit bord de scellage interne proximal auto-expansible (111) et ledit bord de drainage externe distal, dans lequel la longueur du manchon est plus longue que l'épaisseur de l'abdomen, dans lequel ledit manchon (119) comprend une zone qui est auto-aplatissable, en empêchant ainsi un endommagement involontaire des parois de la stomie pendant l'insertion du conduit dans la stomie, et dans lequel la longueur du conduit de drainage de stomie (101) est réglable.

7. Dispositif de drainage de stomie selon la revendication 5, comprenant en outre :
(a) une plaque de base de fixation corporelle (102) pour fixer ledit dispositif stomique à un stomisé, ladite plaque de base (102) comprenant une collerette d'adaptateur de poche pour stomie (105) sans scellant de paroi de stomie externe, conçue pour entourer la paroi externe de la stomie sans contact direct avec elle, dans lequel ladite plaque de base (102) est exempte d'adhésif ou non adhésive, dans lequel ladite plaque de base (102) facultativement : (i) comprend un filtre à gaz (110) avec une couche d'absorption d'odeurs ; ou (ii) est retournable pour permettre une surveillance et une gestion de la peau péristomiale sous la plaque de base, et
(b) une membrane de scellage externe élastique (118) montée sur ledit manchon de raccordement (119), dans lequel un mouvement de ladite membrane externe (118) le long du manchon (119) vers le bord de scellage interne expansible (111) crée une force axiale de scellage pour fixer ledit conduit (101) en place, dans lequel ladite membrane externe (118) est ancrée le long du manchon (119) par des rebords et des saillies (116) dédiés.

8. Dispositif de drainage de stomie selon la revendication 7, comprenant en outre un adaptateur de manchon de scellage souple (118) intégré à ladite plaque de base (102), de telle sorte que ledit bord de drainage externe dudit conduit (101) permet une connexion fluidique scellée entre ledit conduit (101) et une poche pour stomie qui est reliée à ladite collerette d'adaptateur de poche pour stomie (105).

9. Dispositif de drainage de stomie selon la revendication 5, dans lequel le bord de drainage externe dudit conduit (101) comprend une collerette de raccordement détachable (106), et est facultativement équipé d'un bouchon de coiffage (108), dans lequel un manchon de scellage souple (118) est :
- fixé à ladite collerette de raccordement détachable (106) par un clamp ou une bague élastique (107), et
- constitué d'un filtre à membrane hydrophobe perméable au gaz intestinal et imperméable au liquide intestinal.

10. Dispositif de drainage de stomie selon la revendication 5, dans lequel ledit conduit (101) comprend en outre : (i) une endoprothèse de support auto-expansible (120) placé dans celui-ci ; ou (ii) une ou plusieurs bagues de renforcement rigides (115) configurées pour empêcher un aplatissement axial et un retournement du bord de scellage interne expansible (111) pendant l'insertion de celui-ci dans la stomie.

11. Dispositif de drainage de stomie selon la revendication 5, comprenant en outre un applicateur détachable (109) pour insérer le conduit (101) dans la stomie, dans lequel ledit applicateur détachable (109) est auto-aplatissable lorsqu'une force axiale supérieure à 5-10 N est appliquée, en empêchant ainsi une érosion ou une perforation involontaire de l'intestin pendant l'insertion du conduit dans la stomie.

12. Dispositif de drainage de stomie selon la revendication 5, dans lequel la surface extérieure du conduit comprend des sections configurées pour appliquer une force radiale inférieure sur la paroi interne de l'intestin de manière constante ou alternée.

13. Dispositif de drainage de stomie selon la revendication 5, dans lequel le raccord de collerette de poche pour stomie (105) est équipé d'un dispositif de lavage de poche pour stomie comprenant :
a. un tube d'aspersion percé circulaire fixé à la circonférence intérieure du raccord de collerette de la poche pour stomie,
b. un tube de lavage souple relié audit tube d'aspersion, et
c. une buse en caoutchouc souple soudée à l'autre bord dudit tube de lavage flexible et configurée pour s'associer à un embout de robinet.
